# EUROPEAN PATENT APPLICATION

(11) **EP 4 696 689 A1**
(43) Date of publication of application: **18.02.2026**
(21) Application number: 24784347.7
(22) Date of filing: 03.04.2024
(51) Int. Cl.: C07D 471/04, A61K 31/519, A61P 35/00

(54) **SALT FORM AND CRYSTAL FORM OF CHIRAL HETEROCYCLIC COMPOUND HAVING HEDGEHOG PATHWAY ANTAGONIST ACTIVITY**

(30) Priority: 04.04.2023 CN 202310354586
(71) Applicant: Suzhou Kintor Pharmaceuticals, Inc., Jiangsu 215123 (CN)
(72) Inventor: TONG, Youzhi, Suzhou, Jiangsu 215123 (CN)
(74) Representative: Cabinet Becker et Associés
(86) International application number: PCT/CN2024/085809
(87) International publication number: WO 2024/208265

(57) **Abstract**

The present application provides a salt form and crystal form of a chiral heterocyclic compound having hedgehog pathway antagonist activity. The compound is (R)-1-(6-(5'-chloro-3,5-dimethyl-[2,4'-bipyridyl]-2'-yl)-5-methyl-5,6,7,8-tetrahydropyrido[4,3-d]pyrimidin-2-yl)piperidin-4-ol. A pharmaceutically acceptable salt of the compound exhibits a high-temperature endothermic peak and thus has good thermal stability, and the crystal form basically does not change when heated. Particularly, a fumarate anhydrous crystal form F exhibits optimal thermodynamic stability, good physical and chemical stability and medulloblastoma resistance, better hygroscopicity, solubility and bioavailability than free base amorphous forms, and better pharmacokinetic characteristics, a better tumor inhibition effect and stronger hedgehog signal pathway inhibition activity than GDC-0449.

## Description

The present application claims the priority of patent application No. 2023103545860 titled "salt form and crystal form of chiral heterocyclic compound having hedgehog pathway antagonist activity" filed in China on April 4, 2023, the content of which is incorporated herein by reference in its entirety.

### Techinal Field

The present application belongs to the field of drugs, particularly relates to a salt form of a chiral heterocyclic compound (R)-1-(6-(5'-chloro-3,5-dimethyl-[2,4'-bipyridine]-2'-yl)-5-methyl-5,6,7,8-tetrahydropyrido[4,3-d]pyrimidin-2-yl) piperidin-4-ol with hedgehog pathway antagonist activity and a crystal form thereof.

### Background Art

Hedgehog (Hh) pathway is a highly conserved intercellular signal transduction system, which is composed of Hh ligand, two transmembrane protein receptors patched membrane receptor (PTCH) and smoothened transmembrane protein receptor (SMO)and downstream transcription factor Gli protein. There are three Hedgehog homologous genes in human, including sonic hedgehog (SHH), indian hedgehog (IHH) and desert hedgehog (DHH). They are strictly regulated under normal circumstances and play an important role in cell proliferation, cell differentiation and embryonic formation. Under normal circumstances, the Hh signal pathway enters an inactivated state after the embryo matures, and the activity of SMO protein is inhibited. However, if the Hh protein in the signal pathway is abnormally expressed, the inhibitory effect of SMO protein is removed, the Hh signal pathway is abnormally activated, the Gli protein plays a transcriptional activation factor function, triggers the expression of downstream target genes, causes excessive cell proliferation, and finally leads to the occurrence of tumors.

At present, the research of Hh pathway inhibitors mainly targets two important molecules of the signal pathway: one is SMO targeting the upstream of the Hh pathway, and the other is Gli targeting the downstream of the Hh pathway. The development of SMO inhibitors has progressed relatively smoothly and three drugs Vismodegib (GDC-0449, 2012), Sonidegib (LDE225, 2015) and Glasdegib (2018) have been approved by FDA, the former two are used for treating basal cell carcinoma, and the latter is used for treating acute myeloid leukemia. However, it has been found clinically that multiple examples of conformational rearrangement, drug and protein binding site destruction mediated acquired drug resistance cases due to SMO mutations. For example, Vismodegib initiates drug resistance due to the mutation of target SMO D473H and has not been successfully taken in the treatment of medulloblastoma. Therefore, the research and development of efficient novel Hh signaling pathway drugs is still a difficult problem to be solved currently.

WO2018006756A1 discloses a chiral heterocyclic compound with hedgehog pathway antagonist activity, which has the structure shown in the following formula (I).

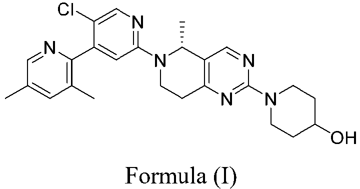

However, according to the preparation method of Example 7 in WO2018006756A1, the prepared compound of formula I is an amorphous compound, and there are some difficulties in drug formulation in the amorphous form of the compound, such as low stability, difficult purification, etc. Therefore, it is necessary to develop a salt form or a crystal compound with excellent stability and physicochemical properties, so as to efficiently inhibit the expression of the Hedgehog signaling pathway, and then effectively inhibit the growth of tumor cells.

### Summary

In view of the problems in the prior art, the present application provides a salt form and a crystal form of the chiral heterocyclic compound (R)-1-(6-(5'-chloro-3,5-dimethyl-[2,4'-bipyridine]-2'-yl)-5-methyl-5,6,7,8-tetrahydropyrido[4,3-d] pyrimidin-2-yl) piperidin-4-ol with hedgehog pathway antagonist activity.

In a first aspect of the present application, the present application provides a pharmaceutically acceptable salt of (R)-1-(6-(5'-chloro-3,5-dimethyl-[2,4'-bipyridin]-2'-yl)-5-methyl-5,6,7,8-tetrahydropyrido[4,3-d] pyrimidin-2-yl)piperidin -4-ol as shown in formula (I):

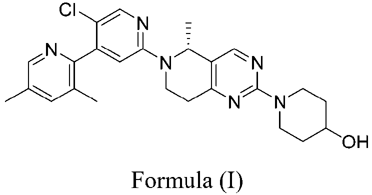

Wherein, the salt includes at least one of fumarate, succinate and hydrochloride.

In a second aspect of the present application, the present application provides a fumarate of the compound of formula (I) in crystal form.

Preferably, the fumarate of the compound of formula (I) in the crystal form of the present application comprises fumarate crystal form A, B, C, D, E, F, G, H of the compound of formula (I).

In a third aspect of the present application, the present application provides succinate of the compound of formula (I) in crystal form.

Preferably, the succinate of the compound of formula (I) in the crystal form of the present application comprises succinate crystal form A, B of the compound of formula (I).

In a fourth aspect of the present application, the present application provides hydrochloride of the compound of formula (I).

In a fifth aspect of the present application, the present application provides a method for preparing a pharmaceutically acceptable salt of the compound of formula (I).

In a sixth aspect of the present application, the present application provides a pharmaceutical composition comprising the pharmaceutically acceptable salt of the compound of formula (I).

In a seventh aspect of the present application, the present application provides an application of the pharmaceutically acceptable salt of the compound of formula (I) in preparation of a drug, and further provides a method for applying the pharmaceutically acceptable salt of the compound of formula (I).

### Effects of the Invention

The present application provides pharmaceutically acceptable salts of (R)-1-(6-(5'-chloro-3,5-dimethyl-[2,4'-bipyridin]-2'-yl)-5-methyl-5,6,7,8-tetrahydropyrido[4,3-d]pyrimidin-2-yl) piperidin-4-ol as shown in formula (I), including fumarate, succinate, hydrochloride, especially for fumarate and succinate crystal form, which includes fumarate crystal form A, B, C, D, E, F, G, H, succinate crystal form A, B. The pharmaceutically acceptable salts of the compound of formula (I) of the present application have a relatively high temperature endothermic peak, and therefore have good thermal stability, and the crystal form does not change substantially when heated. In particular, the fumarate anhydrous crystal form F of the compound of formula (I) shows optimal thermodynamic stability, has good physical and chemical stability, and has excellent anti-tumor, especially anti-myeloblastoma properties; compared with free base amorphous form, the fumarate has relatively small hygroscopicity, higher solubility in water and better bioavailability, and has better pharmacokinetic characteristics, better tumor suppression effect and stronger Hedgehog signal path inhibitory activity compared with the positive drug GDC -0449.

### Brief Description of the Drawings

The drawings of the present application illustrate several embodiments of the present application and, together with the description, serve to explain the principles of the present application. The drawings are for the purpose of illustrating the embodiments of the present application only and are not to be construed as limiting the present application. Other objects, features, and advantages of the present application will become apparent from the following detailed description, taken in conjunction with the drawings, in which illustrative embodiments of the present application are shown, wherein:
FIG. 1 shows an XRPD pattern of succinate crystal form A;
FIG. 2 shows an XRPD pattern of succinate crystal form B;
FIG. 3 shows an XRPD pattern of fumarate crystal form A;
FIG. 4 shows a TGA and DSC diagram of the fumarate crystal form A, wherein a is a TGA graph; b is a DSC graph;
FIG. 5 shows a ¹H NMR graph of the fumarate crystal form A;
FIG. 6 shows an XRPD pattern of the fumarate crystal form B;
FIG. 7 shows a TGA and DSC diagram of the fumarate crystal form B, wherein a is a TGA graph; b is a DSC graph;
FIG. 8 shows a ¹H NMR graph of the fumarate crystal form B;
FIG. 9 shows a variable temperature XRPD pattern of the fumarate crystal form B;
FIG. 10 shows an XRPD pattern of the fumarate crystal form C;
FIG. 11 shows a TGA and DSC diagram of the fumarate crystal form C, wherein a is TGA graph; b is DSC graph;
FIG. 12 shows a ¹H NMR graph of the fumarate crystal form C;
FIG. 13 shows an XRPD pattern of the fumarate crystal form D;
FIG. 14 shows a TGA and DSC diagram of the fumarate crystal form D, wherein a is a TGA graph; b is a DSC graph;
FIG. 15 shows a ¹H NMR graph of the fumarate crystal form D;
FIG. 16 shows an XRPD stack of the fumarate crystal form D before and after drying and heating;
FIG. 17 shows a TGA stack of the fumarate crystal form D before and after drying and heating;
FIG. 18 shows a ¹H NMR stack of the fumarate crystal form D before and after heating;
FIG. 19 shows an XRPD pattern of the fumarate crystal form E;
FIG. 20 shows a TGA and DSC diagram of the fumarate crystal form E, wherein a is a TGA graph; b is a DSC graph;
FIG. 21 shows a ¹H NMR graph of the fumarate crystal form E;
FIG. 22 shows an XRPD stack of the fumarate crystal form E before and after drying;
FIG. 23 shows an XRPD pattern of the fumarate crystal form F;
FIG. 24 shows a TGA and DSC diagram of the fumarate crystal form F, wherein a is a TGA graph; b is a DSC graph;
FIG. 25 shows a ¹H NMR graph of the fumarate crystal form F;
FIG. 26 shows a variable temperature XRPD pattern of the fumarate crystal form F;
FIG. 27 shows an XRPD pattern of the fumarate crystal form G;
FIG. 28 shows a TGA and DSC diagram of the fumarate crystal form G, wherein a is a TGA graph; b is a DSC graph;
FIG. 29 shows a ¹H NMR graph of the fumarate crystal form G;
FIG. 30 shows an XRPD pattern of the fumarate crystal form H;
FIG. 31 shows a TGA and DSC diagram of the fumarate crystal form H, wherein a is a TGA graph; b is a DSC graph;
FIG. 32 shows a ¹H NMR graph of the fumarate crystal form H;
FIG. 33 shows an XRPD stack of the fumarate crystal form H before and after drying and heating;
FIG. 34 shows a TGA stack of the fumarate crystal form H before and after drying and heating;
FIG. 35 shows an XRPD stack (I/II) of a suspension competitive sample;
FIG. 36 shows an XRPD stack (II/II) of a suspension competitive sample;
FIG. 37 shows a DVS graph of the amorphous free base;
FIG. 38 shows an XRPD stack of the amorphous free base before and after DVS testing;
FIG. 39 shows a DVS graph of the fumarate crystal form F;
FIG. 40 shows an XRPD stack of the fumarate crystal form F before and after DVS testing;
FIG. 41 shows a dynamic solubility curve;
FIG. 42 shows an XRPD stack of solubility samples of the amorphous free base in H₂O;
FIG. 43 shows an XRPD stack of solubility samples of the amorphous free base in SGF;
FIG. 44 shows an XRPD stack of solubility samples of the amorphous free base in FaSSIF;
FIG. 45 shows an XRPD stack of solubility samples of t the amorphous free base in FeSSIF;
FIG. 46 shows an XRPD stack of solubility samples of fumarate crystal form F in H₂O;
FIG. 47 shows an XRPD stack of solubility samples of fumarate crystal form F in SGF;
FIG. 48 shows an XRPD stack of solubility samples of fumarate crystal form F in FaSSIF;
FIG. 49 shows an XRPD stack of solubility samples of fumarate crystal form F in FeSSIF;
FIG. 50 shows an XRPD stack of stability assessment samples of the amorphous free base;
FIG. 51 shows an XRPD stack of stability assessment samples of fumarate crystal form F;
FIG. 52 shows the body weight change curve of SCID mice bearing subcutaneous tumors during the treatment and drug withdrawal observation period;
FIG. 53 shows the comparison of the tumor inhibition efficacy of GDC-0449 and compound A in SCID mice bearing subcutaneous tumors;
FIG. 54 shows the relative tumor volume change curve during the treatment period in SCID mice bearing subcutaneous tumors;
FIG. 55 shows the relative tumor volume change curve during the treatment and drug withdrawal observation period in SCID mice bearing subcutaneous tumors;
FIG. 56 shows the comparison of differences in antitumor efficacy between compound A and GDC-0449;
FIG. 57 shows the comparison of differences in tumor recurrence during the drug withdrawal observation period between compound A and GDC-0449;
FIG. 58 shows the comparison of mRNA expression levels of Gli1 gene in tumor tissues of each treatment group.

Before proceeding with the detailed description, it is to be appreciated that the following detailed description is merely exemplary in nature and is not intended to limit the invention or the application and uses thereof. Hence, although the present disclosure is, for convenience of explanation, depicted and described as shown in certain illustrative embodiments, it will be appreciated that it can be implemented in various other types of embodiments and equivalents, and in various other systems and environments. Furthermore, there is no intention to be bound by any theory presented in the preceding background or the following detailed description.

### Detailed Description of the Invention

Unless otherwise defined, the terms used herein have the same meaning as commonly understood by one of ordinary skill in the art. The terms used in the specification are only used to describe particular embodiments and are not intended to limit the present application.

Unless the context indicates or implies otherwise, the terms used herein have the meanings defined below. Unless otherwise prohibited or implied, for example, by including mutually exclusive elements or options, in those definitions and throughout this specification, the terms "a" and "an" mean one or more and the term "or" means and/or where the context permits. Thus, as presented in the specification and the appended claims, the singular forms "a", "an" and "the" include plural referents unless the context clearly dictates otherwise.

At various locations in the present disclosure, e.g., in any disclosed embodiments or in the claims, reference is made to compounds, compositions, or methods that "comprise" one or more specified components, elements or steps. Invention embodiments also specifically include those compounds, compositions combinations, or methods that are, or that consist of, or that consist essentially of those specified components, elements or steps. For example, compositions, devices, articles disclosed as "comprising" a component or step are open-ended, and they include or read on those compositions or methods plus an additional component(s) or step(s). However, those terms do not encompass unrecited elements that would destroy the functionality of the disclosed compositions, devices, articles of manufacture or methods for its intended purpose. Similarly, disclosed compositions, devices, articles of manufacture or methods that "consist of" a component or step are closed, and they would not include or read on those compositions or methods having appreciable amounts of an additional component(s) or an additional step(s). Furthermore, the term "consisting essentially of" admits for the inclusion of unrecited elements that have no material effect on the functionality of the disclosed compositions, devices, articles of manufacture or methods for its intended purpose as further defined herein.

"About", as the term is used herein, unless otherwise stated or implied by context, in connection with a numeric value or range of values to describe a particular property of a compound or composition, indicate that the value or range of values may deviate to an extent deemed reasonable to one of ordinary skill in the art while still describing the particular property. Reasonable deviations include those that are within the accuracy or precision of the instrument(s) used in measuring, determining or deriving the particular property. Specifically, the term "about" when used in this context, indicate that the numeric value or range of values can vary by 10%, 9%, 8%, 7%, 6%, 5%, 4%, 3%, 2%, 1 %, 0.9%, 0.8%, 0.7%, 0.6%, 0.5%, 0.4%, 0.3%, 0.2%, 0.1 %, or 0.01% of the recited value or range of values, typically by 10% to 0.5 %, more typically by 5% to 1%, while still describing the particular property.

As used herein, unless the context indicates or specifies, the symbol "±" is used to indicate that the preceding value is subject to the error or deviation of the following value. Specifically, the expression "X±Y" (where X and Y are both numerical values) means that the error or deviation of the value X is Y, that is, (X-Y) to (X+Y) are within the range of X±Y.As used herein, unless the context indicates or specifies, the tilde "~" is used to indicate a numerical range that is inclusive of the numerical values stated before and after it. Specifically, the expression "X to Y" (where X and Y are both numerical values) means "greater than X and less than Y".

As used herein, unless the context indicates or specifies, the term "substantially as shown" when referring to, for example, an XRPD pattern/chart, TGA pattern, DSC pattern, etc., refers to patterns/values that are not necessarily the same as those described herein, but that fall within the limits of experimental error or deviation when considered by one of ordinary skill in the art.

As used herein, unless the context indicates or specifies, the term "h" is a unit of time, meaning hour.

As used herein, unless the context indicates or specifies, the term "d" is a unit of time, meaning day.

As used herein, unless the context indicates or specifies, "room temperature" specifically refers to 23 ± 3°C.

Compounds are generally described herein using standard nomenclature. For compounds with asymmetric centers, it should be understood that (unless otherwise stated) includes all optical isomers and mixtures thereof. Furthermore, compounds with carbon-carbon double bonds may be present in the Z-type and E-type, unless stated otherwise, all isomeric forms of the compounds are included in the present application. When a compound exists in various tautomeric forms, the enumerated compound is not limited to any particular tautomer but is intended to include all tautomeric forms.

In a first aspect of the present application, the present application provides a pharmaceutically acceptable salt of (R)-1-(6-(5'-chloro-3,5-dimethyl-[2,4'-bipyridin]-2'-yl)-5-methyl-5,6,7,8-tetrahydropyrido[4,3-d] pyrimidin-2-yl)piperidin -4-ol as shown in formula (I):

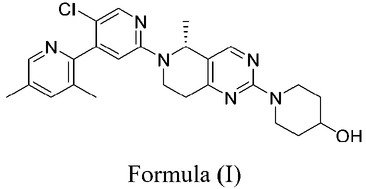

Wherein, the salt includes at least one of fumarate, succinate, hydrochloride.

The present application finds that the compound of formula (I) are quite difficult to form salts with acids, particularly to obtain salts in solid form. The present application further finds that the compound of formula (I) can obtain a stable solid form or even a crystal form under certain specific reaction conditions with fumaric acid, succinic acid or hydrochloric acid, which contributes to the application of the compound of formula (I).

Thus, in some embodiments, the pharmaceutically acceptable salt of the compound of formula (I) comprises at least one of fumarate, succinate and hydrochloride of the compound of formula (I). Preferably, the pharmaceutically acceptable salt is fumarate of the compound of formula (I).

In some embodiments, in the fumarate salt, the molar ratio of the compound of formula (I) to the fumaric acid is 1: 0.2~1, preferably 1: 0.2, 1: 0.3, 1: 0.4, 1: 0.5, 1: 0.6, 1: 0.7, 1: 0.8, 1: 0.9, 1: 1.0; further preferably 1: 0.3~0.6, more preferably 1: 0.5.

In a second aspect of the present application, the present application provides a fumarate of the compound of formula (I) in crystal form.

In one aspect, the present application provides a fumarate crystal form A of the compound of formula (I).

The fumarate crystal form A of the compound of formula (I) provided in the present application has X-ray powder diffraction pattern comprising characteristic peaks at 20 values of 6.0±0.2°, 12.9±0.2°, and 16.2±0.2°; furthermore, the fumarate crystal form A of the compound of formula (I) provided in the present application has X-ray powder diffraction pattern further comprising characteristic peaks at one or two or three of 2θ values of 13.3 ± 0.2°, 15.4 ± 0.2°, 19.0 ± 0.2°; preferably, the fumarate crystal form A of the compound of formula (I) provided in the present application has X-ray powder diffraction pattern further comprising characteristic peaks at one or two or three of 20 values of 12.2 ± 0.2°, 18.1 ± 0.2°, 20.7 ± 0.2°; more preferably, the fumarate crystal form A of the compound of formula (I) provided in the present application has X-ray powder diffraction pattern comprising characteristic peaks at 20 values of 6.0 ± 0.2°, 6.5 ± 0.2°, 9.9 ± 0.2°, 12.2 ± 0.2°, 12.9 ± 0.2°, 13.3 ± 0.2°, 13.7 ± 0.2°, 15.4 ± 0.2°, 16.2 ± 0.2°, 17.6 ± 0.2°, 18.1 ± 0.2°, 19.0 ± 0.2°, 19.6 ± 0.2°, and 20.7 ± 0.2°.

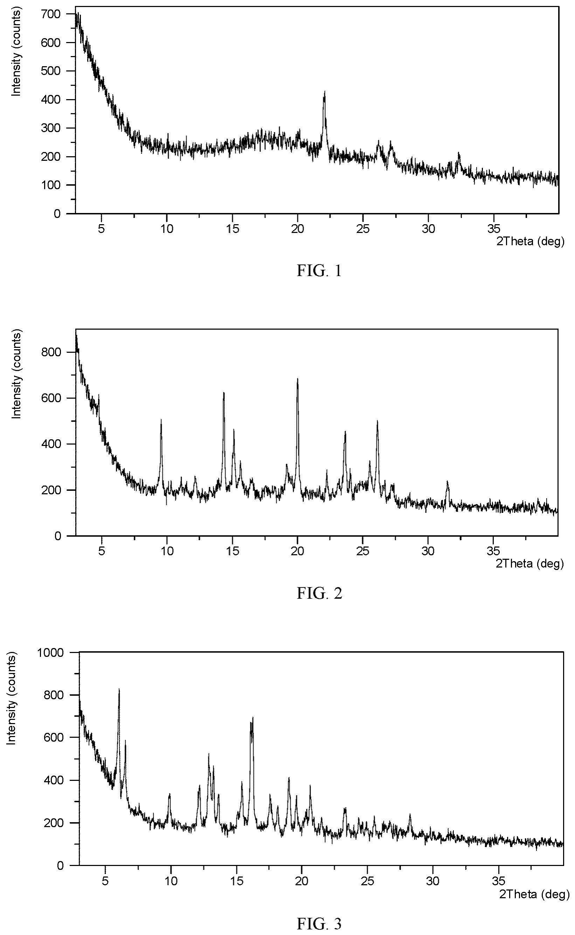

Furthermore, the fumarate crystal form A of the compound of formula (I) provided in the present application has an X-ray powder diffraction pattern substantially as shown in FIG.3, and/or has diffraction peaks substantially as shown in Table 2-1.

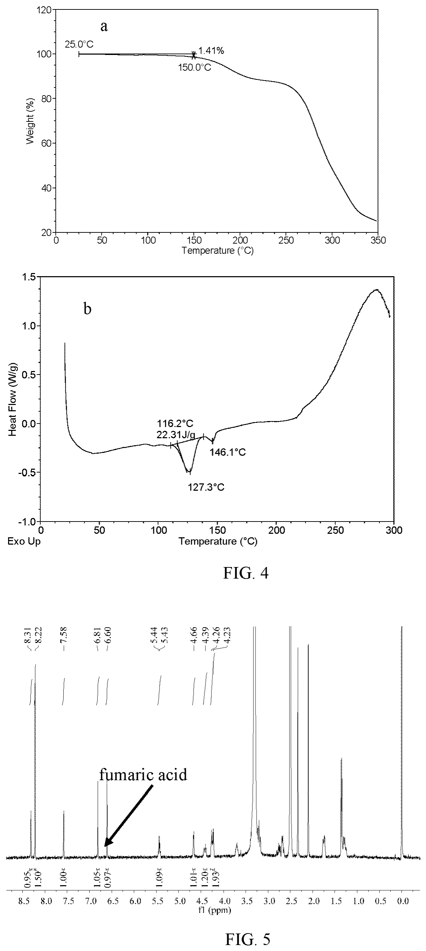

The fumarate crystal form A of the compound of formula (I) provided in the present application, when subjected to thermogravimetric analysis, has a weight loss gradient of about 1.4 ± 0.5% (eg, 1.4 ± 0.2%) when heated to 150°C. Preferably, the thermogravimetric analysis diagram is basically as shown in FIG. 4.a.

The fumarate crystal form A of the compound of formula (I) provided in the present application has an endothermic peak at 127.3 ± 2°C (eg, 127.3 ± 1°C) and 146.1 ± 2°C (eg, 146.1 ± 1°C) when performing differential scanning calorimetry analysis. Preferably, the differential scanning calorimetry analysis diagram is basically as shown in FIG. 4.b.

The fumarate crystal form A of the compound of formula (I) provided in the present application, wherein the molar ratio of the compound of formula (I) to fumaric acid is 1: 0.5.

The fumarate crystal form A of the compound of formula (I) provided in the present application is anhydrous compound.

The present application further provides a method for preparing the fumarate crystal form A of the compound of formula (I), the method comprising: stirring the compound of formula (I) and fumaric acid in a mixed solvent of an alkyl ketone and an alkane, filtering and drying to obtain the fumarate crystal form A.

Furthermore, the molar ratio of the compound of formula (I) to the fumaric acid in the preparation method is 1: 0.3~1.5, preferably 1: 0.3, 1: 0.4, 1: 0.5, 1: 0.6, 1: 0.7, 1: 0.8, 1: 0.9, 1: 1.0, 1: 1.5, and more preferably 1: 0.5~0.7.

Furthermore, the volume ratio of the alkyl ketone to the alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5.

Furthermore, the alkyl ketone may comprise acetone, butanone, methylisobutylketone and cyclohexanone; the alkane may comprise isopentane, n-pentane, cyclopentane, n-hexane, cyclohexane, n-heptane, isooctane, and petroleum ether; the mixed solvent of the alkyl ketone and the alkane is preferably a mixed solvent of acetone and n-heptane.

Furthermore, the temperature during stirring is -5~40°C, preferably 5~35°C, more preferably room temperature.

Furthermore, the stirring is performed under a dark condition.

Furthermore, the stirring time is 1h~8d, preferably 6h~5d, and more preferably 12h~1d.

Furthermore, the mixture may be stirred at room temperature for 1~4d and then stirred at 0-10°C for 1~4d.

In another aspect, the present application provides a fumarate crystal form B of the compound of formula (I).

The fumarate crystal form B of the compound of formula (I) provided in the present applicationhas has X-ray powder diffraction pattern comprising characteristic peaks at 20 values of 13.2±0.2°, 16.0±0.2°, 18.6±0.2°; furthermore, the fumarate crystal form B of the compound of formula (I) provided in the present application has X-ray powder diffraction pattern further comprising characteristic peaks at one or two or three of 12.7 ± 0.2°, 15.4 ± 0.2°, 16.6 ± 0.2°; preferably, the fumarate crystal form B of the compound of formula (I) provided in the present application has X-ray powder diffraction pattern further comprising characteristic peaks at one or two or three of 17.2 ± 0.2°, 18.0 ± 0.2°, 20.4 ± 0.2°; more preferably, the fumarate crystal form B of the compound of formula (I) provided in the present application has X-ray powder diffraction pattern comprising characteristic peaks at 20 values of 12.7 ± 0.2°, 13.2 ± 0.2°, 15.4 ± 0.2°, 16.0 ± 0.2°, 16.6 ± 0.2°, 17.2 ± 0.2°, 18.0 ± 0.2°, 18.6 ± 0.2°, 19.0 ± 0.2°, 20.4 ± 0.2°, 22.8 ± 0.2°, 26.8 ± 0.2°, 28.8 ± 0.2°, 29.4 ± 0.2° or has X-ray powder diffraction comprising characteristic peaks at 20 values of 12.7 ± 0.2°, 13.2 ± 0.2°, 15.4 ± 0.2°, 15.8 ± 0.2°, 16.0 ± 0.2°, 16.6 ± 0.2°, 17.2 ± 0.2°, 18.0 ± 0.2°, 18.6 ± 0.2°, 19.0 ± 0.2°, 20.4 ± 0.2°, 22.8 ± 0.2°, 26.8 ± 0.2°, 28.8 ± 0.2°, 29.4 ± 0.2°.

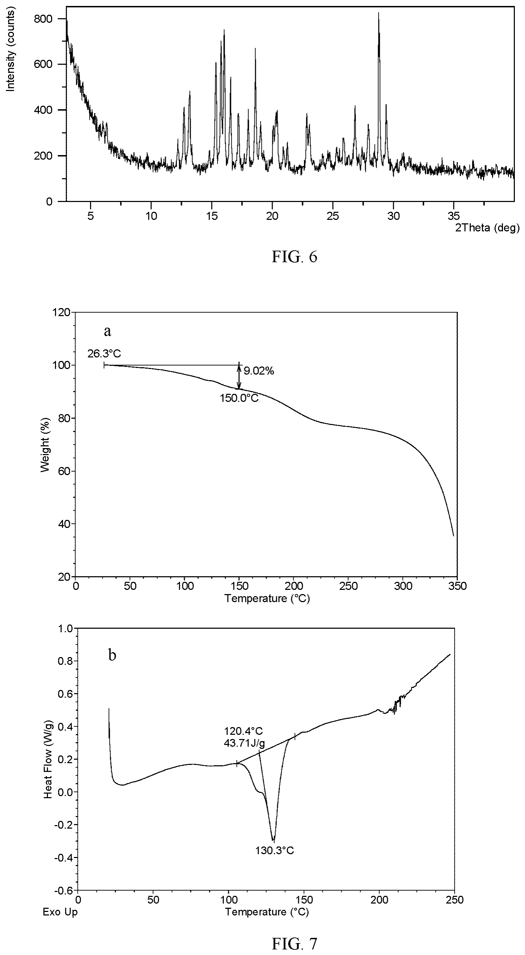

Furthermore, the fumarate crystal form B of the compound of formula (I) provided in the present application has X-ray powder diffraction pattern substantially as shown in FIG. 6, and/or has a diffraction peak substantially as shown in Table 2-2.

The fumarate crystal form B of the compound of formula (I) provided in the present application, when subjected to thermogravimetric analysis, has a weight loss gradient of about 9.0 ± 0.5% (eg, 9.0 ± 0.2%) when heated to 150°C. Preferably, the thermogravimetric analysis diagram is basically as shown in FIG. 7.a.

The fumarate crystal form B of the compound of formula (I) provided in the present application has an endothermic peak at 130.3 ± 2°C (eg, 130.3 ± 1°C) when performing differential scanning calorimetry analysis. Preferably, the differential scanning calorimetry analysis diagram is basically as shown in FIG. 7 b.

The fumarate crystal form B of the compound of formula (I) provided in the present application, wherein the molar ratio of the compound of formula (I) to the fumaric acid is 1: 0.6.

The fumarate crystal form B of the compound of formula (I) provided in the present application is anhydrous compound.

The present application further provides a method for preparing the fumarate crystal form B of the compound of formula (I), the method comprising: stirring the compound of formula (I) and fumaric acid in a mixed solvent of an ester and an alkane, filtering, and drying to obtain the fumarate crystal form B.

Furthermore, the molar ratio of the compound of formula (I) to the fumaric acid in the preparation method is 1: 0.3 -1.5, preferably 1: 0.3, 1: 0.4, 1: 0.5, 1: 0.6, 1: 0.7, 1: 0.8, 1: 0.9, 1: 1.0, 1: 1.5, and more preferably 1: 0.5~0.7.

Furthermore, the volume ratio of the ester to the alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5.

Furthermore, the ester may comprise ethylacetate and isopropylacetate; the alkane may comprise isopentane, n-pentane, cyclopentane, n-hexane, cyclohexane, n-heptane, isooctane, and petroleum ether; the mixed solvent of the ester and the alkane is preferably a mixed solvent of ethylacetate and cyclohexane.

Furthermore, the temperature during stirring is 5~40°C, preferably 15~35°C, more preferably room temperature.

Furthermore, the stirring is performed under a dark condition.

Furthermore, the stirring time is 0.5~10d, preferably 1~8d, more preferably 1~4d.

In another aspect, the present application provides a fumarate crystal form C of the compound of formula (I).

The fumarate crystal form C of the compound of formula (I) provided in the present application has X-ray powder diffraction pattern comprising characteristic peaks at 20 values of 15.1 ± 0.2°, 17.1 ± 0.2°, 19.4 ± 0.2°; furthermore, the fumarate crystal form C of the compound of formula (I) provided in the present application has X-ray powder diffraction pattern further comprising characteristic peaks at one or two or three of 11.0 ± 0.2°, 17.8 ± 0.2°, 21.7 ± 0.2°; preferably, the fumarate crystal form C of the compound of formula (I) provided in the present application has X-ray powder diffraction pattern further comprising characteristic peaks at one or two or three of 5.0 ± 0.2°, 10.1 ± 0.2°, 27.1 ± 0.2°; more preferably, the fumarate crystal form C of the compound of formula (I) provided in the present application has X-ray powder diffraction pattern comprising characteristic peaks at 20 values of 5.0 ± 0.2°, 10.1 ± 0.2°, 11.0 ± 0.2°, 12.9 ± 0.2°, 15.1 ± 0.2°, 16.1 ± 0.2°, 17.1 ± 0.2°, 17.8 ± 0.2°, 19.4 ± 0.2°, 20.2 ± 0.2°, 21.7 ± 0.2°, 27.1 ± 0.2°.

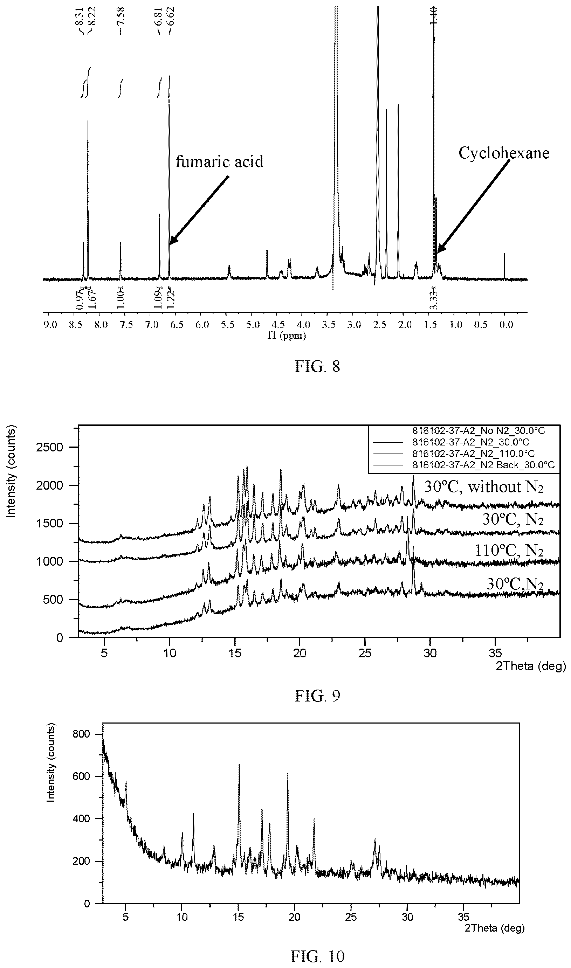

Furthermore, the fumarate crystal form C of the compound of formula (I) provided in the present application has an X-ray powder diffraction pattern substantially as shown in FIG.10, and/or has diffraction peaks substantially as shown in Table 2-3.

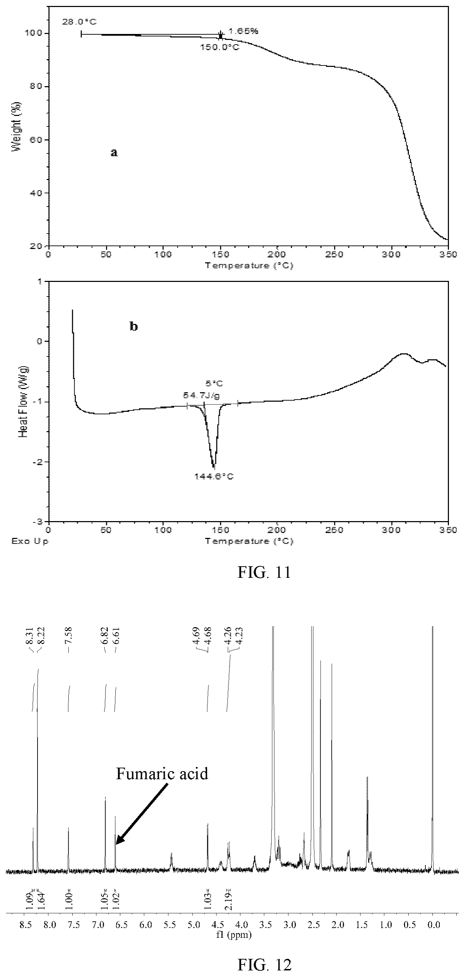

The fumarate crystal form C of the compound of formula (I) provided in the present application, when subjected to thermogravimetric analysis, has a weight loss gradient of about 1.7±0.5% (eg, 1.7±0.2%) when heated to 150°C. Preferably, the thermogravimetric analysis diagram is basically as shown in FIG. 11.a.

The fumarate crystal form C of the compound of formula (I) provided in the present application has an endothermic peak at 144.6 ± 2°C (eg, 144.6 ± 1°C) when performing differential scanning calorimetry analysis. Preferably, the differential scanning calorimetry analysis diagram is basically as shown in FIG. 11 b.

The fumarate crystal form C of the compound of formula (I) provided in the present application, wherein the molar ratio of the compound of formula (I) to the fumaric acid is 1: 0.5.

The fumarate crystal form C of the compound of formula (I) provided in the present application is anhydrous compound.

The present application further provides a method for preparing the fumarate crystal form C of the compound of formula (I), the method comprising: stirring a compound of formula (I) and fumaric acid, or a fumarate salt of the compound of formula (I) in a linear or branched C2 -C6 nitrile, filtering, and drying to obtain the fumarate crystal form C.

Furthermore, the fumarate salt of the compound of formula (I) comprises the fumarate crystal form A of the compound of formula (I).

Furthermore, the linear or branched C2 -C6 nitrile may comprise acetonitrile.

Furthermore, the temperature during stirring is -5~40°C, preferably 5~30°C, preferably room temperature.

Furthermore, the stirring is performed under a dark condition.

Furthermore, the stirring time is 6h~12d, preferably 10h~8d, and more preferably 12h ~ 6d.

Furthermore, when the compound of formula (I) and the fumaric acid are used as raw materials, stirring may be performed at room temperature for 6~18 h, and then stirred at 0~10°C for 10~20 h.

In another aspect, the present application provides a fumarate crystal form D of the compound of formula (I).

The fumarate crystal form D of the compound of formula (I) provided in the present application has X-ray powder diffraction pattern comprising characteristic peaks at 20 values of 12.7 ± 0.2°, 17.6 ± 0.2°, 19.1 ± 0.2°; furthermore, the fumarate crystal form D of the compound of formula (I) provided in the present application has X-ray powder diffraction pattern further comprising characteristic peaks at one or two or three of 6.3 ± 0.2°, 13.2 ± 0.2°, 16.1 ± 0.2°; preferably, the fumarate crystal form D of the compound of formula (I) provided in the present application has X-ray powder diffraction pattern further comprising characteristic peaks at one or two or three of 17.2 ± 0.2°, 14.0 ± 0.2°, 20.9 ± 0.2°; more preferably, the fumarate crystal form D of the compound of formula (I) provided in the present application has characteristic peaks at 2θ values of 6.3 ± 0.2°, 11.3 ± 0.2°, 12.7 ± 0.2°, 13.2 ± 0.2°, 14.0 ± 0.2°, 16.1 ± 0.2°, 17.2 ± 0.2°, 17.6 ± 0.2°, 19.1 ± 0.2°, 20.5 ± 0.2°, 20.9 ± 0.2°, and 24.1 ± 0.2°.

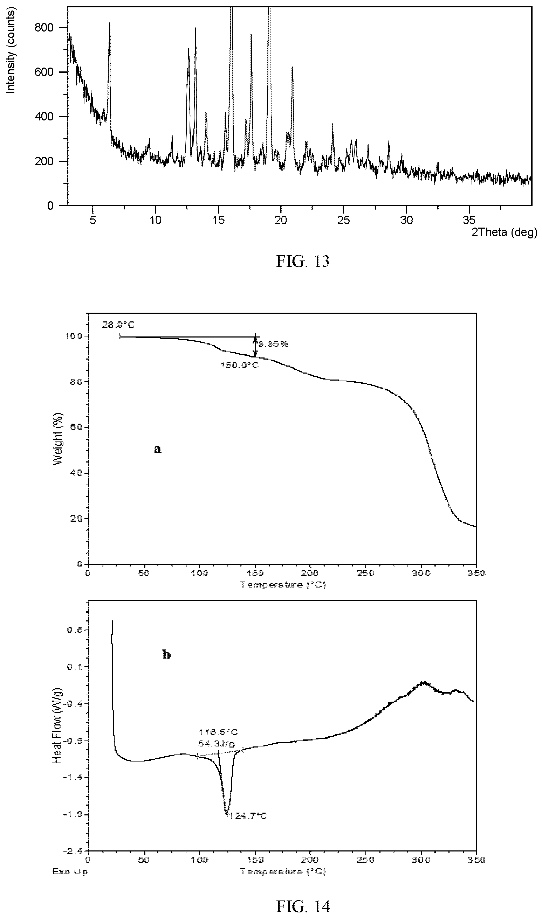

Furthermore, the fumarate crystal form D of the compound of formula (I) provided in this application has X-ray powder diffraction pattern substantially as shown in FIG. 13, and/or has a diffraction peak substantially as shown in Table 2-4.

The fumarate crystal form D of the compound of formula (I) provided in the present application, when subjected to thermogravimetric analysis, has a weight loss gradient of about 8.9 ± 0.5% (eg, 8.9 ± 0.2%) when heated to 150°C. Preferably, the thermogravimetric analysis diagram is basically as shown in FIG. 14. a.

The fumarate crystal form D of the compound of formula (I) provided in the present application has an endothermic peak at 124.7 ± 2°C (eg, 124.7 ± 1°C) when performing differential scanning calorimetry analysis. Preferably, the differential scanning calorimetry analysis diagram is basically as shown in FIG. 14 b.

The fumarate crystal form D of the compound of formula (I) provided in the present application, wherein the molar ratio of the compound of formula (I) to the fumaric acid is 1: 0.5.

The present application further provides a method for preparing the fumarate crystal form D of the compound of formula (I), the method comprising: stirring a fumarate salt of the compound of formula (I) in ether, or a mixed solvent of a cyclicamide and an aromatic hydrocarbon, filtering, and drying to obtain the fumarate crystal form D.

Furthermore, the fumarate salt of the compound of formula (I) comprises the fumarate crystal form A of the compound of formula (I).

Furthermore, the ether may comprise methyltert-butylether, tetrahydrofuran or 2-methyltetrahydrofuran, preferably methyltert-butylether; the cyclicamide may include N-methylpyrrolidone, and the aromatic hydrocarbon may include benzene, toluene, xylene; the mixed solvent of the cyclicamide and the aromatic hydrocarbon is preferably a mixed solvent of N-methylpyrrolidone and toluene.

Furthermore, the temperature during stirring is-5~40°C, preferably 0~30°C, more preferably 0~10°C, or room temperature.

Furthermore, the volume ratio of the cyclic amide to the aromatic hydrocarbon is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, the stirring is performed under a dark condition.

Furthermore, the stirring time is 1~15 d, preferably 3~10 d, more preferably 5~8 d.

In another aspect, the present application provides a fumarate crystal form E of the compound of formula (I).

The fumarate crystal form E of the compound of formula (I) provided in the present application has X-ray powder diffraction pattern comprising characteristic peaks at 20 values of 15.2 ± 0.2°, 16.0 ± 0.2°, 22.0 ± 0.2°; furthermore, the fumarate crystal form E of the compound of formula (I) provided in the present application has X-ray powder diffraction pattern further comprising characteristic peaks at one or two or three of 12.8 ± 0.2°, 20.5 ± 0.2°, 26.0 ± 0.2°; preferably, the fumarate crystal form E of the compound of formula (I) provided in the present application has X-ray powder diffraction pattern further comprising characteristic peaks at one or two or three of 16.8 ± 0.2°, 18.1 ± 0.2°, 19.7 ± 0.2°; more preferably, the fumarate crystal form E of the compound of formula (I) provided in the present application has X-ray powder diffraction pattern comprising characteristic peaks at 20 values of 12.8 ± 0.2°, 15.2 ± 0.2°, 16.0 ± 0.2°, 16.8 ± 0.2°, 18.1 ± 0.2°, 19.7 ± 0.2°, 20.5 ± 0.2°, 22.0 ± 0.2°, 23.9 ± 0.2°, 26.0 ± 0.2°.

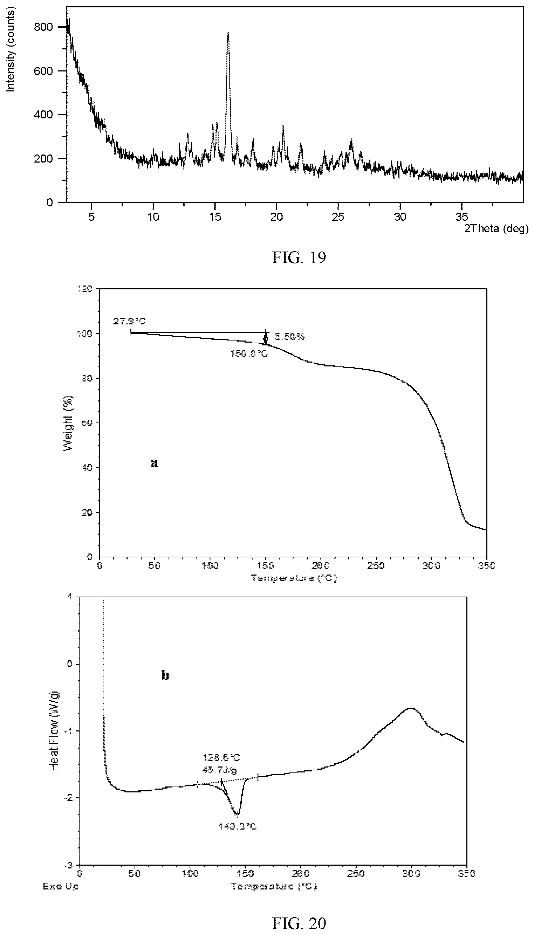

Furthermore, the fumarate crystal form E of the compound of formula (I) provided in the present application has X-ray powder diffraction pattern substantially as shown in FIG. 19, and/or has a diffraction peak substantially as shown in Table 2-5.

The fumarate crystal form E of the compound of formula (I) provided in the present application, when subjected to thermogravimetric analysis, has a weight loss gradient of about 5.5 ± 0.5% (eg, 5.5 ± 0.2%) when heated to 150°C. Preferably, the thermogravimetric analysis diagram is basically as shown in FIG. 20. a.

The fumarate crystal form E of the compound of formula (I) provided in the present application has an endothermic peak at 143.3 ± 2°C (eg, 143.3 ± 1°C) when performing differential scanning calorimetry analysis. Preferably, the differential scanning calorimetry analysis diagram is basically as shown in FIG. 20 b.

The fumarate crystal form E of the compound of formula (I) provided in the present application, wherein the molar ratio of the compound of formula (I) to the fumaric acid is 1: 0.5.

The fumarate crystal form E of the compound of formula (I) provided in the present application is hydrate or anhydrous crystal form that is hygroscopic (surface water adsorption).

The present application also provides a method for preparing the fumarate crystal form E of the compound of formula (I), the method comprising: stirring a fumarate salt of a compound of formula (I) in a mixed solvent of an alkylalcohol and an alkane, filtering, and drying to obtain the fumarate crystal form E.

Furthermore, the fumarate salt of the compound of formula (I) comprises the fumarate crystal form A of the compound of formula (I).

Furthermore, the alkylalcohol may comprise methanol, ethanol, n-propanol, and isopropanol; the alkane may comprise isopentane, n-pentane, cyclopentane, n-hexane, cyclohexane, n-heptane, isooctane and petroleum ether; the mixed solvent of the alkylalcohol and the alkane is preferably a mixed solvent of isopropanol and n-heptane.

Furthermore, the volume ratio of the alkyl alcohol to the alkane is 1: 1~6, preferably 1: 1, 1: 2, 1: 3, 1: 4, 1: 5, 1: 6, more preferably 1: 3~5.

Furthermore, the temperature during stirring is 15~40°C, preferably room temperature.

Furthermore, the stirring is performed under a dark condition.

Furthermore, the stirring time is 1~10 d, preferably 2~8 d, more preferably 3~6 d.

In another aspect, the present application provides a fumarate crystal form F of the compound of formula (I).

The fumarate crystal form F of the compound of formula (I) provided in the present application has X-ray powder diffraction pattern comprising characteristic peaks at 20 values of 11.1 ± 0.2°, 16.6 ± 0.2°, 22.4 ± 0.2°; furthermore, the fumarate crystal form F of the compound of formula (I) provided in the present application has X-ray powder diffraction pattern further comprising characteristic peaks at one or two or three of 16.9 ± 0.2°, 18.6 ± 0.2°, 21.0 ± 0.2°; preferably, the fumarate crystal form F of the compound of formula (I) provided in the present application has X-ray powder diffraction pattern further comprising characteristic peaks at one or two or three of 5.5 ± 0.2°, 14.5 ± 0.2°, 25.0 ± 0.2°; more preferably, the fumarate crystal form F of the compound of formula (I) provided in the present application has characteristic peaks at 2θ values of 5.5 ± 0.2°, 11.1 ± 0.2°, 14.5 ± 0.2°, 15.5 ± 0.2°, 16.6 ± 0.2°, 16.9 ± 0.2°, 17.5 ± 0.2°, 18.6 ± 0.2°, 21.0 ± 0.2°, 22.4 ± 0.2°, 23.3 ± 0.2°, 25.0 ± 0.2°.

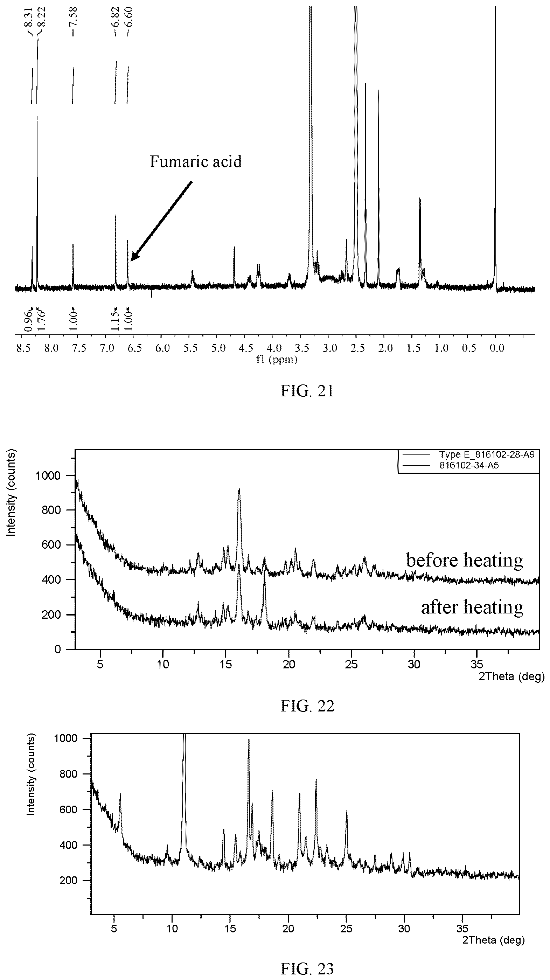

Furthermore, the fumarate crystal form F of the compound of formula (I) provided in the present application has X-ray powder diffraction pattern substantially as shown in FIG. 23, and/or has a diffraction peak substantially as shown in Table 2-6.

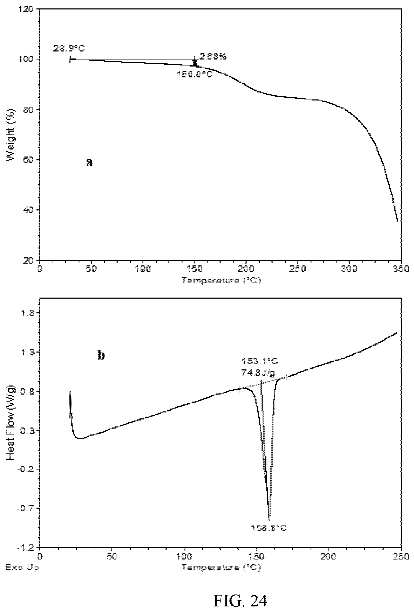

The fumarate crystal form F of the compound of formula (I) provided in the present application, when subjected to thermogravimetric analysis, has a weight loss gradient of about 2.7 ± 0.5% (eg, 2.7 ± 0.2%) when heated to 150°C. Preferably, the thermogravimetric analysis diagram is basically as shown in FIG. 24 a.

The fumarate crystal form F of the compound of formula (I) provided in the present application has an endothermic peak at 158.8 ± 2°C (eg, 158.8 ± 1°C) when performing differential scanning calorimetry analysis. Preferably, the differential scanning calorimetry analysis diagram is basically as shown in FIG. 24 b.

The fumarate crystal form F of the compound of formula (I) provided in the present application, wherein the molar ratio of the compound of formula (I) to the fumaric acid is 1: 0.5.

The fumarate crystal form F of the compound of formula (I) provided in the present application is anhydrous compound.

The present application further provides a method for preparing the fumarate crystal form F of the compound of formula (I), wherein the method is selected from any one of the following:
i. stirring the compound of formula (I) and fumaric acid in an organic solvent, filtering and drying to obtain;
ii. stirring the fumarate of the compound of formula (I) in an organic solvent, filtering and drying to obtain.

Furthermore, in method i, the molar ratio of the compound of formula (I) to the fumaric acid is 1: 0.3~1.5, preferably 1: 0.3, 1: 0.4, 1: 0.5, 1: 0.6, 1: 0.7, 1: 0.8, 1: 0.9, 1: 1.0, 1: 1.5, and more preferably 1: 0.5~0.7.

Furthermore, in method i, the organic solvent comprises a mixed solvent of alkylketone and alkane, C2 -C6 nitrile, ether, a mixed solvent of alkyl alcohol and alkane, and a mixed solvent of alkylalcohol and water.

Furthermore, the alkyl ketone may comprise acetone, butanone, methylisobutylketone, and cyclohexanone; the linear or branched C2-C6 nitrile may comprise acetonitrile; the ether may comprise methyltert-butylether, tetrahydrofuran, 2-methyltetrahydrofuran, 1,4-dioxane, preferably methyltert-butylether; the alkylalcohol may comprise methanol, ethanol, n-propanol, isopropanol; the alkane may comprise isopentane, n-pentane, cyclopentane, n-hexane, cyclohexane, n-heptane, isooctane, and petroleum ether; the mixed solvent of the alkylketone and the alkane is preferably a mixed solvent of acetone and n-heptane; the mixed solvent of the alkylalcohol and the alkane is preferably a mixed solvent of isopropanol and n-heptane; and the mixed solvent of the alkylalcohol and the water is preferably a mixed solvent of ethanol and water.

Furthermore, in the mixed solvent of alkylketone and alkane, the volume ratio of alkyl ketone to alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 2~5; in the mixed solvent of alkylalcohol and alkane, the volume ratio of alkylalcohol to alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5; in a mixed solvent of alkylalcohol and water, the volume ratio of alkylalcohol to water is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, in method i, in the case of using the mixed solvent, the order of adding compound of formula (I), fumaric acid, and solvent is not limited. For example, after the organic solvent forming the mixed solvent can be prepared into a mixed solvent in proportion, the compound of formula (I) and fumaric acid are added, or the compound of formula (I) and fumaric acid can also be added to one of the solvents forming the mixed solvent, and then the rest of the solvent is added.

Furthermore, in method i, the temperature during stirring is 0~70°C, preferably 15°C, 20°C, 25°C, 30°C 35°C 40°C, 45°C, 50°C, 55°C, 60°C, 65°C, 70°C, more preferably room temperature, or 45~55°C.

Furthermore, in method i, stirring is performed under a dark condition.

Furthermore, in method i, the stirring time is 6 h~9 d, preferably 6 h, 12 h, 1 d, 2 d, 3 d, 4 d, 5 d, 6 d, 7 d, 8 d, 9 d, and more preferably 2~6 d.

Furthermore, in method ii, the fumarate salt of the compound of formula (I) comprises a fumarate crystal form A, a crystal form B, or a crystal form C.

Furthermore, in method ii, the organic solvent is selected from alkylketone, C2-C6 nitrile, and ether; furthermore, the alkyl ketone may comprise acetone, butanone, methyl isobutyl ketone, cyclohexanone, preferably acetone, methyl isobutylketone; the C2-C6 nitrile may comprise acetonitrile; the ether may comprise methyltert-butylether, tetrahydrofuran, 2-methyltetrahydrofuran, 1,4-dioxane, preferably methyltert-butylether;

Furthermore, in method ii, the temperature during stirring is 15~70°C., preferably 15°C, 20°C, 25°C, 30°C, 35°C, 40°C, 45°C, 50°C, 55°C, 60°C, 65°C, 70°C more preferably room temperature, or 45~55°C.

Furthermore, in method ii, stirring is performed under a dark condition.

Furthermore, in method ii, the stirring time is 0.5~12d, preferably 0.5d, 1d, 2d, 3d, 4d, 5d, 6d, 7d, 8d, 9d, 10d, 11d, 12d, more preferably 0.5~8d.

In another aspect, the present application provides a fumarate crystal form G of the compound of formula (I).

The fumarate crystal form G of the compound of formula (I) provided in the present application has X-ray powder diffraction pattern comprising characteristic peaks at 20 values of 15.6 ± 0.2°, 17.7± 0.2°, 20.6± 0.2°.

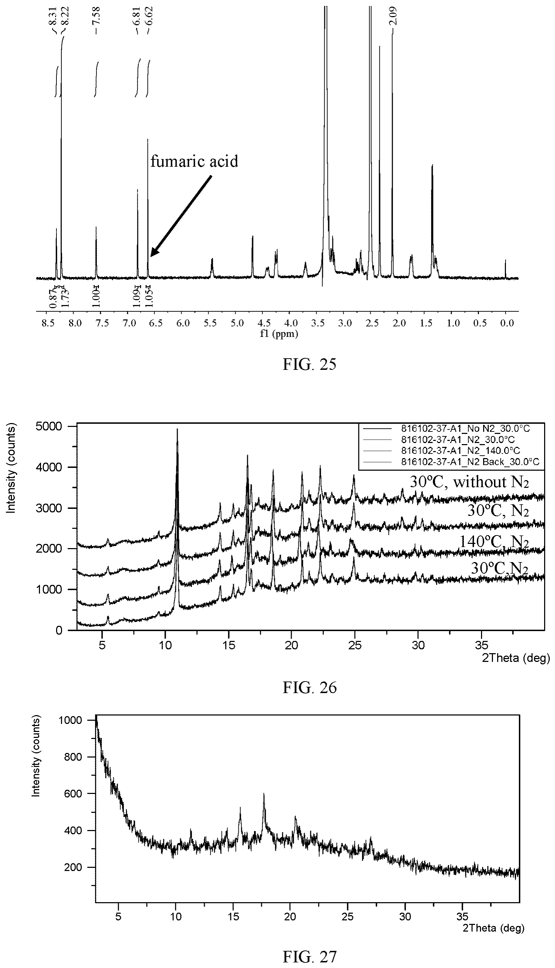

Furthermore, the fumarate crystal form G of the compound of formula (I) provided in the present application has X-ray powder diffraction pattern substantially as shown in FIG. 27, and/or has a diffraction peak substantially as shown in Table 2-9.

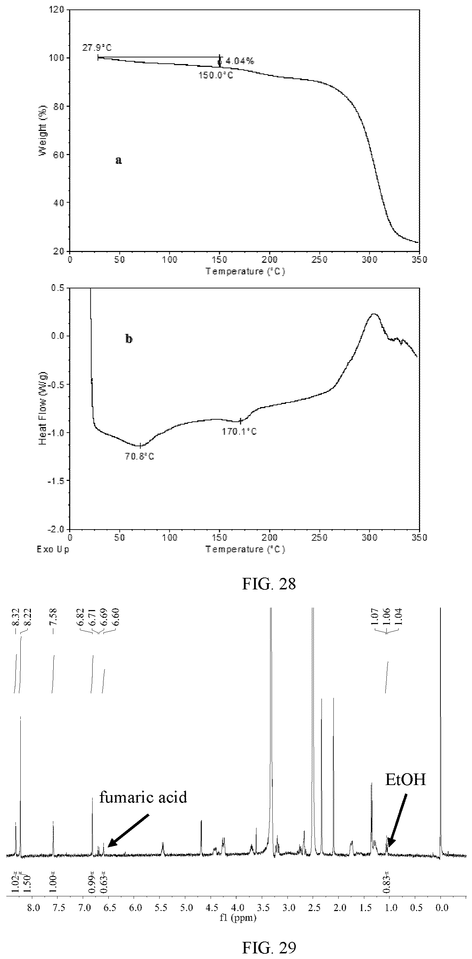

The fumarate crystal form G of the compound of formula (I) provided in the present application, when subjected to thermogravimetric analysis, has a weight loss gradient of about 4.0 ± 0.5% (eg, 4.0 ± 0.2%) when heated to 150°C. Preferably, the thermogravimetric analysis diagram is basically as shown in FIG. 28 a.

The fumarate crystal form G of the compound of formula (I) provided in the present application has an endothermic peak at 70.8 ± 2°C (eg, 70.8 ± 1°C) and 170.1 ± 2°C, eg, 170.1 ± 1°C) when performing differential scanning calorimetry analysis. Preferably, the differential scanning calorimetry analysis diagram is basically as shown in FIG. 28 b.

The fumarate crystal form G of the compound of formula (I) provided in the present application, wherein the molar ratio of the compound of formula (I) to the fumaric acid is 1: 0.3.

The present application further provides a method for preparing the fumarate crystal form G of a compound of formula (I), the method comprising: stirring a fumarate salt of the compound of formula (I) in a mixed solvent of alkylalcohol and water, or a mixed solvent of a cyclicamide and water, filtering, and drying to obtain.

Furthermore, the fumarate salt of the compound of formula (I) comprises fumarate crystal form A of the compound of formula (I).

Furthermore, the alkylalcohol may comprise methanol, ethanol, n-propanol, isopropanol, preferably ethanol; the cyclicamide may comprise N-methylpyrrolidone; the mixed solvent of the alkylalcohol and the water is preferably a mixed solvent of ethanol and water; and the mixed solvent of the cyclicamide and the water is preferably a mixed solvent of N-methylpyrrolidone and water.

Furthermore, the volume ratio of the alkyl alcohol to water is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, the volume ratio of the cyclicamide to water is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, the temperature during stirring is 15~70°C, preferably 45~55°C.

Furthermore, the stirring is performed under a dark condition.

Furthermore, the stirring time is 1~10 d, preferably 2~8 d, more preferably 3~7 d.

In another aspect, the present application provides a fumarate crystal form H of the compound of formula (I).

The fumarate crystal form H of the compound of formula (I) provided in the present application has X-ray powder diffraction pattern comprising characteristic peaks at 20 values of 12.4 ± 0.2°, 16.2 ± 0.2°, 21.8 ± 0.2°; furthermore, the fumarate crystal form H of the compound of formula (I) provided in the present application has X-ray powder diffraction pattern further comprising characteristic peaks at one or two or three of 12.8 ± 0.2°, 15.8 ± 0.2°, 18.0 ± 0.2°; preferably, the fumarate crystal form H of the compound of formula (I) provided in the present application has X-ray powder diffraction pattern further comprising characteristic peaks at one or two or three of 14.5 ± 0.2°, 16.9 ± 0.2°, 21.2 ± 0.2°; more preferably, the fumarate crystal form H of the compound of formula (I) provided in the present application has characteristic peaks at 2θ values of 5.2 ± 0.2°, 12.4 ± 0.2°, 12.8 ± 0.2°, 14.5 ± 0.2°, 15.8 ± 0.2°, 16.2 ± 0.2°, 16.9 ± 0.2°, 18.0 ± 0.2°, 20.3 ± 0.2°, 21.2 ± 0.2°, 21.8 ± 0.2°, 23.7 ± 0.2°.

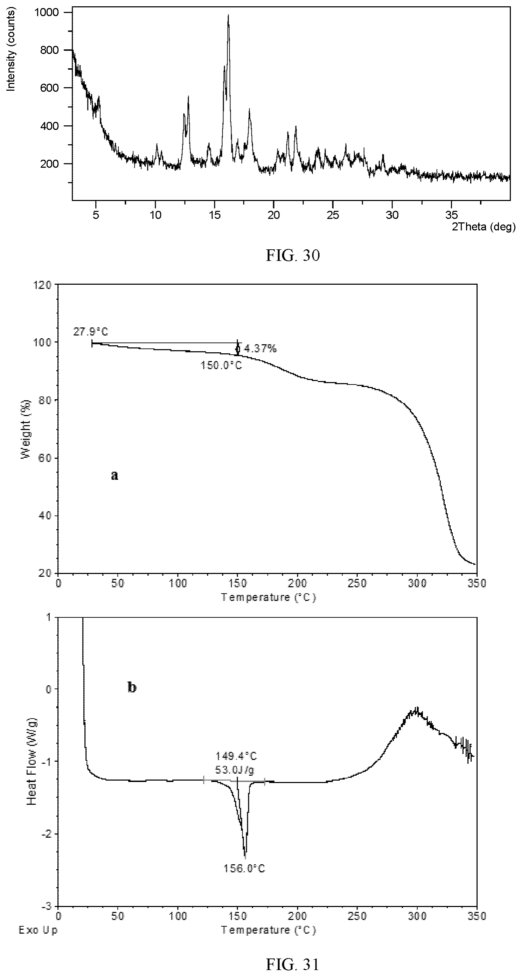

Furthermore, the fumarate crystal form H of the compound of formula (I) provided in the present application, the X-ray powder diffraction pattern thereof is substantially as shown in FIG. 30, and/or has a diffraction peak substantially as shown in Table 2-10.

The fumarate crystal form H of the compound of formula (I) provided in the present application, when subjected to thermogravimetric analysis, has a weight loss gradient of about 4.4 ± 0.5% (eg, 4.4 ± 0.2%) when heated to 150°C. Preferably, the thermogravimetric analysis diagram is basically as shown in FIG. 31 a.

The fumarate crystal form H of the compound of formula (I) provided in the present application has an endothermic peak at 156.0 ± 2°C (eg, 156.0 ± 1°C) when performing differential scanning calorimetry analysis. Preferably, the differential scanning calorimetry analysis diagram is basically as shown in FIG. 31 b.

The fumarate crystal form H of the compound of formula (I) provided in the present application, wherein the molar ratio of the compound of formula (I) to the fumaric acid is 1: 0.5.

The fumarate crystal form H of the compound of formula (I) provided in the present application is hydrate or anhydrous crystal form that is hygroscopic (surface water adsorption).

The present application further provides a method for preparing the fumarate crystal form H of a compound of formula (I), the method comprising: stirring a fumarate salt of a compound of formula (I) in a mixed solvent of an alkylalcohol and an alkane, filtering and drying to obtain.

Furthermore, the fumarate salt of the compound of formula (I) comprises the fumarate crystal form A of the compound of formula (I).

Furthermore, the alkylalcohol may comprise methanol, ethanol, n-propanol, and isopropanol; the alkane may comprise isopentane, n-pentane, cyclopentane, n-hexane, cyclohexane, n-heptane, isooctane, and petroleum ether; and the mixed solvent of the alkylalcohol and the alkane is preferably a mixed solvent of isopropanol and n-heptane.

Furthermore, the volume ratio of the alkylalcohol to the alkane is 1: 7~15, preferably 1: 7, 1: 8, 1: 9, 1: 10, 1: 11, 1: 12, 1: 13, 1: 14, 1: 15, more preferably 1: 7~11.

Furthermore, the temperature during stirring is 15~70°C, preferably 45~55°C.

Furthermore, the stirring is performed under a dark condition.

Furthermore, the stirring time is 1~10 d, preferably 2~8 d, more preferably 3~7 d.

In a third aspect of the present application, the present application provides a succinate of the compound of formula (I) in crystal form.

In one aspect, the present application provides a succinate crystal form A of the compound of formula (I).

The succinate crystal form A of the compound of formula (I) provided in the present application has X-ray powder diffraction pattern comprising characteristic peaks at 20 values of 22.1 ± 0.2° and 27.2 ± 0.2°.

Furthermore, the succinate crystal form A of the compound of formula (I) provided in the present application has X-ray powder diffraction pattern thereof comprising diffraction peaks substantially as shown in Table 1-3.

The present application further provides a method for preparing the succinate crystal form A of the compound of formula (I), the method comprising: stirring the compound of formula (I) and succinic acid in an ether, filtering and drying to obtain.

Furthermore, the ether may comprise methyltert-butylether, tetrahydrofuran, 2-methyltetrahydrofuran, 1,4-dioxane, preferably methyltert-butylether.

Furthermore, the molar ratio of the compound of formula (I) to succinic acid is 1: 0.3~3.0, preferably 1: 0.5~2.0, more preferably 1: 0.8~1.2.

Furthermore, the reaction conprises stirring the compound of formula (I) and succinic acid in methyl tert-butyl ether, stirring at 0~10°C for 1~4 days, stirring for 1~3 days at -25~15°C, and then volatilizing at room temperature.

Furthermore, the stirring is performed under a dark condition.

In one aspect, the present application provides a succinate crystal form B of the compound of formula (I).

The succinate crystal form B of the compound of formula (I) provided in the present application has X-ray powder diffraction pattern comprising characteristic peaks at 2θ values of 9.5 ± 0.2°, 14.4 ± 0.2°, 23.6 ± 0.2°; furthermore, the succinate crystal form B of the compound of formula (I) provided in the present application has X-ray powder diffraction pattern further comprising characteristic peaks at one or two or three of 15.1 ± 0.2°, 19.2± 0.2°, 25.5 ± 0.2°; preferably, the succinate crystal form B of the compound of formula (I) provided in the present application has X-ray powder diffraction pattern further comprising characteristic peaks at one or two or three of 15.6 ± 0.2°, 22.3 ± 0.2°, 24.0 ± 0.2°; more preferably, the succinate crystal form B of the compound of formula (I) provided in the present application has X-ray powder diffraction pattern comprising characteristic peaks at 20 values of 9.5 ± 0.2°, 12.2 ± 0.2°, 14.4 ± 0.2°, 15.1 ± 0.2°, 15.6 ± 0.2°, 16.5 ± 0.2°, 19.2 ± 0.2°, 22.3 ± 0.2°, 23.6 ± 0.2°, 24.0 ± 0.2°, 25.5 ± 0.2°, 27.3 ± 0.2°.

Furthermore, the succinate crystal form B of the compound of formula (I) provided in the present application has X-ray powder diffraction pattern thereof comprising diffraction peaks substantially as shown in Table 1-4.

The present application further provides a method for preparing the succinate crystal form B of the compound of formula (I), the method comprising: stirring the compound of formula (I) and succinic acid in a mixed solvent of alkylketone and alkane, filtering and drying to obtain.

Furthermore, the alkylketone may comprise acetone, butanone, methylisobutylketone, and cyclohexanone; the alkane comprises isopentane, n-pentane, cyclopentane, n-hexane, cyclohexane, n-heptane, isooctane, and petroleum ether; and the mixed solvent of the alkylketone and the alkane is preferably a mixed solvent of acetone and n-heptane.

Furthermore, the molar ratio of the compound of formula (I) to succinic acid is 1: 0.3~3.0, preferably 1: 0.5~2.0, more preferably 1: 0.8~1.2.

Furthermore,the volume ratio of the alkyl ketone to the alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5.

Furthermore, the temperature during stirring is 15~40°C, preferably room temperature.

Furthermore, the stirring is performed under a dark condition.

Furthermore, the stirring time is 1~8 d, preferably 2~7 d, and more preferably 4~6 d.

In a fourth aspect of the present application, the present application provides a hydrochloride of the compound of formula (I).

The present application further provides a method for preparing the hydrochloride of the compound of formula (I), the method comprising: the compound of formula (I) and HCl were stirred in an organic solvent, filtered and dried to obtain.

Furthermore, the organic solvent is an ester, which may comprise ethylacetate, isopropyl acetate, preferably ethylacetate.

In this application, the mixed solvent only means that it is a mixed solvent during the reaction process but does not mean that it must be used in the form of a mixed solvent. In this application, there are no restrictions on the use of mixed solvents. For example, the organic solvent forming the mixed solvent can be mixed in proportion to form a mixed solvent for use; or one or more solvents forming the mixed solvent can be added to the reactor first, and then the remaining solvent is added.

In this application, the process of forming the crystal further comprises adding a seed crystal for stirring.

The present application provides a pharmaceutical composition comprising the pharmaceutically acceptable salt of the compound of formula (I).

Therefore, the present application provides a pharmaceutical composition comprising a pharmaceutically acceptable salt of the compound of formula (I), and a pharmaceutically acceptable carrier.

The present application further provides a pharmaceutical composition comprising fumarate, succinate and hydrochloride of the compound of formula (I) and other pharmaceutically acceptable salts of the compound of formula (I).

The present application further provides a combined application composition 1, comprising: (i) any one or moreof cisplatin, paclitaxel, camptothecin, trastuzumab, glider, imatinib, gefitinib, erlotinib, and lapatinib; and (ii) the pharmaceutically acceptable salts of the compound of formula (I), which are simultaneously or sequentially used, ie, administered separately or separately within a certain time interval.

The present application further provides another conbined application composition 2, comprising: (i) N-acetylcysteine, pirfenidone, nintedanib, glucocorticoids (such as methylprednisolone, prednisone, fluticasone), immunosuppressants (such as cyclosporine, cyclophosphamide, Azathioprine), and endothelin receptor antagonists (such as Macitentan, Bosentan, Ambrisentan ), JNK Inhibitors ( Tanzisertib (CC -930)), Itraconazole, Vismodegib, hydroxycarbamide, Danazol, Amikacin, Pulmozyme, Tobramycin,Tiotropium bromide, Treprostinil, Zileuton, Saridegib ( IPI -926 ); and ( ii ) a pharmaceutically acceptable salt of the compound of formula ( I ), which are simultaneously or sequentially used, ie, administered separately or separately within a certain time interval.

In some embodiments, the pharmaceutically acceptable salts of the compound of formula (I) are used to treat subjects by administering the compounds as pharmaceutical compositions. For this purpose, in one embodiment, the compound is combined with one or more pharmaceutically acceptable excipients (including carriers, diluents or adjuvants) to form a suitable composition, which is described in more detail herein.

As used herein, the term "excipient" refers to any pharmaceutically acceptable additive, carrier, adjuvant, or other suitable component other than the active pharmaceutical ingredient (API), which is typically included for formulation and/or application purposes. The "diluent" and "adjuvant" are defined below.

As used herein, the terms "treatment" "adminster treatment", "treatment effect" and "therapy" refer to therapy, including but not limited to, curative therapy, preventative treatment, and prophylactic treatment. Preventative treatment generally constitutes the onset of a disease-preventing onset or an onset of a preclinical disease phase of a delayed individual.

The phrase "effective amount" is intended to quantify the amount of each agent that will achieve the goal of improving the severity of the condition and the frequency of occurrence of each agent's own treatment while avoiding adverse side effects typically associated with alternative therapies. In one embodiment, the effective amount is administered in a single dosage form or in multiple dosage forms.

Regardless of the route of administration, the compound of the present application (which may be used in suitable hydrated form) and/or the pharmaceutical composition of the present application is formulated to make an optically acceptable dosage form or by other methods known to those skilled in the art.

The actual dose level of the active ingredient in the pharmaceutical composition of the present application can be varied to obtain an effective amount of the active ingredient to achieve the desired therapeutic response to a particular patient, composition and prescription in the absence of toxicity to the patient.

The selected dose level will depend on a variety of factors, including the activity, route of administration, administration time, excretion rate of the particular compound used, treatment duration, other drugs, compounds and/or materials used in conjunction with the particular hedgehog inhibitor used, age, gender, weight, condition of the patient, general health conditions, and factors known in the art of medical art such as medical history.

A physician or veterinarian having ordinary skill in the art can readily confirm and prescribe an effective amount of the desired pharmaceutical composition. For example, a doctor or veterinarian may prescribe the dosage of the compound of the present application used in the pharmaceutical composition at a level lower than required to achieve the desired therapeutic effect, and gradually increase the dose until the desired effect is achieved.

In general, a suitable daily dose of a pharmaceutically acceptable salt of the compound of formula (I) of the present application is the amount of compound, which is the lowest dose effective to produce a therapeutic effect. This effective dose typically depends on the factors described above. Typically, the disclosed compound of the present application is administered via intravenous, intracerebroventricular, or subcutaneous routes, with a daily dose of 0.0001~100 mg/kg. The method of administration has a significant impact on the dosage. Higher dosage may be used for local delivery approaches.

If necesary, the effective daily dose of the active compound may be administered in any of the following ways: as a single dose, or as two, three, four, five, six or more sub-doses administered at appropriate intervals throughout the day. Those skilled in the art will readily appreciate that the dose level may vary depending on the particular compound, severity of symptoms, and sensitivity of the subject to side effects. Those skilled in the art may readily determine the dose of a given compound disclosed herein by various methods.

In some embodiments, the compounds described herein are formulated into pharmaceutical compositions. The pharmaceutical composition is formulated in a conventional manner using one or more pharmaceutically acceptable inactive ingredients that facilitate the processing of the active compound into a pharmaceutically acceptable formulation. The appropriate recipe depends on the selected route of administration. An overview of the pharmaceutical compositions described herein can be seen, for example, in Remington: The Science and Practice of Pharmacy, nineItemEnth Ed, Easton, Pa. : Mack Publishing Company (1995); Hoover, John E, Remington 's Pharmaceutical Sciences, Mack Publishing Co, Easton, Pa. (1975); Liberman, H A and Latchman, L, Eds. , Pharmaceutical Dosaige Forms, Arachiocelecker, New York, N Y (1980); and Pharmaceutical Dosaige Forms and Drug Eliverylazy Steams, Selength Ed, Lippinitt Williams & Wilkins (1999), the disclosure of which is incorporated herein by reference.

As used herein, a pharmaceutical composition refers to a mixture of pharmaceutically acceptable salts of the compound of formula (I) with other chemical components (pharmaceutically acceptable inactive ingredients) such as carriers, excipients, binders, fillers, suspending agents, flavoring agents, sweeteners, disintegrants, dispersants, surfactants, lubricants, colorants, diluents, solubilizing agents, wetting agents, plasticizers, stabilizers, penetration enhancers, wetting agents, defoamers, antioxidants, preservatives, or one or more combinations thereof. The pharmaceutical composition facilitates the administration of a compound to an organism. In implementing the therapeutic methods or uses provided herein, a therapeutically effective amount of a compound described herein is administered in the form of a pharmaceutical composition to a mammal suffering from a disease, disorder, or condition to be treated. In some embodiments, the mammal is a human. The therapeutically effective amount may vary widely depending on the severity of the disease, the age and relative health of the subject, the pharmacodynamic activity of the compound used, and other factors. The compound may be used alone or in combination with one or more therapeutic agents as a mixture component.

The pharmaceutical formulations described herein are administered to a subject via an appropriate route of administration include, but not limited to, oral, parenteral (eg, intravenous, subcutaneous, intramuscular), intranasal, oral, topical, rectal, or transdermal administration routes. The pharmaceutical formulations described herein, including but are not limited to aqueous liquid dispersions, self-emulsifying dispersions, solid solutions, liposome dispersions, aerosols, solid dosage forms, powders, immediate release formulations, controlled release formulations, fast melt formulations, tablets, capsules, pills, delayed release formulations, extended release formulations, pulse release formulations, multi-particle formulations, and mixed immediate release and controlled release formulations.

All formulations for oral administration are in a dose suitable for such administration. Examples of such dosage units are tablets or capsules. In certain embodiments, these substances contain about 1 to 2000 milligrams of active ingredient, advantageously functional between about 1 to 500 milligrams, and typically between 5 and 150 milligrams. For humans or other mammals, suitable daily doses vary widely depending on the patient's condition and other factors, however, conventional methods and practices may be used again to determine.

Conventional formulation techniques include, for example, a method or combination of methods: (1) dry blending, (2) direct compression, (3) milling, (4) dry or non-aqueous granulation, (5) wet granulation, or (6) fusion. Other methods include, for example, spray drying, pot coating, melt granulation, granulation, fluidized bed spray drying or coating (eg, Wurster coating), tangential coating, top spraying, tabletting, extrusion, etc.

Suitable carriers for the solid dosage forms described herein include, but not limited to, arabic gum, gelatin, colloidal silica, calcium glycerophosphate, calcium lactate, maltodextrin, glycerol, magnesium silicate, sodium caseinate, soybean lecithin, sodium chloride, tricalcium phosphate, dipotassium phosphate, sodium stearoyl lactate, carrageenan, monoglyceride, diglyceride, pregelatinized starch, hydroxypropyl methylcellulose, hydroxypropyl methyl cellulose acetate, sucrose, microcrystalline cellulose, lactose, mannitol, and the like.

Suitable fillers for the solid dosage forms described herein include, but are not limited to, lactose, calcium carbonate, calcium phosphate, calcium hydrophosphate, calcium sulfate, microcrystalline cellulose, cellulose powder, dextrose, glucose binding agent, glucan, starch, pregelatinized starch, hydroxypropyl methylcellulose (HPMC), hydroxypropyl methyl cellulose phthalate, hydroxypropyl methyl cellulose acetate stearate (HPMCAS), sucrose, xylitol, lactitol, mannitol, sorbitol, sodium chloride, polyethylene glycol, and the like.

Suitable disintegrants for use in the solid dosage forms described herein include, but are not limited to, natural starches, such as corn starch or potato starch, pregelatinized starch, or sodium starch glycolate; cellulose, such as methyl crystalline cellulose, methylcellulose, microcrystalline cellulose, croscarmellose; or cross-linked cellulose, such as croscarmellose sodium, crosslinked carboxymethyl cellulose, or crosslinked carboxycellulose; cross-linked starch, such as sodium starch glycolate; cross-linked polymers such as cross-linked povidone, crosslinked polyvinylpyrrolidone; alginate, such as alginic acid or alginic acid salts such as sodium alginate; tree gums such as agar, guar gum, robinia pseudoacacia, phoenix tree, pectin or milkvetch gum; sodium starch glycolate, bentonite, sodium dodecyl sulfate, sodium dodecyl sulfate in the combined starch, and the like.

The adhesive imparts cohesion to the solid oral dosage form formulation: for the powder-filled capsule formulation, they help to form a plug that can be filled into the soft shell or hard shell capsule, and for the tablet formulation, they ensure that the tablet remains intact after compression and helps to ensure the mixing uniformity prior to the compression or filling step. Suitable materials for use as binders in solid dosage forms described herein include, but are not limited to, carboxymethyl cellulose, methylcellulose, hydroxypropyl methylcellulose, hydroxypropyl methyl cellulose acetate, hydroxyethyl cellulose, hydroxypropyl cellulose, ethylcellulose and microcrystalline cellulose, microcrystalline dextrose, linear starch, magnesium aluminosilicate, polysaccharide acid, bentonite, gelatin, polyvinyl pyrrolidone/vinyl acetate copolymer, cross-linked povidone, polyvidone, starch, pregelatinized starch, milkvetch gum, dextrin, sugar (such as sucrose, glucose, dextrose, molasses, mannitol, sorbitol, xylitol, lactose), natural or synthetic gums (such as gum arabic, milkvetch gum, Indian gum, Isakol skin mucus), polyvinylpyrrolidone, larch arabinogalactan, polyethylene glycol, wax, sodium alginate, and the like.

Typically, 20~70% of the adhesive level is used in the powder-filled gelatin capsule formulation. Regardless of the direct tabletting, wet granulation, rolling, or other excipients (eg, the filler itself may be used as a medium adhesive), the level of adhesive usage in the tablet formulation is different. It is common to have an adhesive content of up to 70% in tablet formulations.

Suitable lubricants or glidants for the solid dosage forms described herein include, but are not limited to, stearic acid, calcium hydroxide, talc, corn starch, sodium stearoyl fumarate, alkali metal and alkaline earth metal salts (eg, aluminum salts, calcium salts, magnesium salts, zinc salts), sodium stearate, magnesium stearate, zinc stearate, wax, Stearwet ^{®}, boric acid, sodium benzoate, sodium acetate, sodium chloride, leucine, polyethylene glycol or methoxy polyethylene glycol such as Carbowax ^{™}, PEG 4000, PEG 5000, PEG 6000, propylene glycol, sodium oleate, glycerol behenate, glyceryl palmitate, glycerol benzoate, magnesium lauryl sulfate, sodium lauryl sulfate, and the like.

Suitable diluents for the solid dosage forms described herein include, but are not limited to, sugars (including lactose, sucrose and dextrose), polysaccharides (including dextran and maltodextrin), polyols (including mannitol, xylitol and sorbitol), cyclodextrin, and the like.

Suitable wetting agents for use in the solid dosage forms described herein include, for example, oleic acid, glyceryl monostearate, sorbitan monooleate, sorbitan monolaurate, triethanolamine oleate, polyoxyethylene sorbitan monooleate, polyoxyethylene sorbitan monolaurate, quaternary ammonium compounds (eg, Polyquat 10), sodium oleate, sodium dodecyl sulfate, magnesium stearate, docusate sodium, triacetin, vitamin E TPGS, etc.

Suitable surfactants for use in the solid dosage forms described herein include, for example, sodium lauryl sulfate, sorbitan monooleate, polyoxyethylene sorbitan monooleate, polysorbate, polaxomers, bile salts, glyceryl monostearate, ethylene oxide, and propylene oxide copolymers, such as Pluronic ^{®} (BASF), etc.

Suitable suspending agents for use in the solid dosage forms described herein include, but are not limited to, polyvinylpyrrolidone, such as polyvinylpyrrolidone K12, polyvinylpyrrolidoneK17, polyvinylpyrrolidone K25 or polyvinylpyrrolidone K30, polyethylene glycol, such as polyethylene glycol may have a molecular weight of from about 300 to about 6000, or from about 3350 to about 4000, or from about 7000 to about 5400, vinylpyrrolidone/vinyl acetate copolymer (S630), sodium carboxymethyl cellulose, methylcellulose, hydroxypropyl methylcellulose, polysorbate-80, hydroxyethyl cellulose, sodium alginate, gums, such as Astragalus gum and gum arabic, guar gum, xanthan gum, including xanthan gum, sugar, cellulose, such as sodium carboxymethyl cellulose, methylcellulose, , hydroxypropyl methylcellulose, hydroxyethyl cellulose, polysorbate-80, sodium alginate, polyethoxylated sorbitan monolaurate, povidone, and the like.

In one aspect of the present application, the present application provides the use of the pharmaceutically acceptable salt of the compound of formula (I) and the pharmaceutical composition in the preparation of a drug, the drug being used for treating a disease caused by abnormal activation of a hedgehog signaling pathway; preferably, the disease caused by abnormal activation of the hedgehog signaling pathway is a tumor or interstitial pneumonia.

In one aspect of the present application, the present application provides a pharmaceutically acceptable salt of the compound of formula (I), the pharmaceutical composition, and a combined application composition 1 in preparation of a drug, wherein the drug is used for anti-tumor; preferably, the tumors include: basal cell carcinoma, breast cancer, cervical cancer, colon cancer, rectal cancer, glioma, hepatocellular carcinoma, leukemia, lung cancer, lymphoma, neuroblastoma, multiple myeloma, oral cancer, ovarian cancer, pancreatic cancer, prostate cancer, gastric c ancer, upper GI cancer, esophageal cancer, nasopharyngeal carcinoma, skin cancer, bone cancer, kidney cancer, sarcoma, and brain cancer.

In one aspect of the present application, the present application provides a pharmaceutically acceptable salt of the compound of formula (I), the pharmaceutical composition, and a combined application composition 2 in preparation of a drug, wherein the drug is used for treating interstitial pneumonia; preferably, the interstitial pneumonia is preferably idiopathic interstitial pneumonia, such as idiopathic pulmonary fibrosis, desquamative interstitial pneumonia, non-specific interstitial pneumonia, cryptic pneumonia, respiratory bronchiolitis-related interstitial lung diseases, lymphocyte interstitial pneumonia, and further preferably idiopathic pulmonary fibrosis.

In one aspect of the present application, the present application provides a method for inhibiting hedgehog pathway activation, and further provides a method for treating a disease caused by abnormal activation of hedgehog signaling pathway, and the present application further provides a method for anti-tumor, the method comprising administering to a mammalian subject a therapeutically effective amount of at least one of the pharmaceutically acceptable salt of the compound of formula (I), the pharmaceutical composition described in the present application, or the combined application composition 1.

In one aspect of the present application, the present application provides a method for inhibiting hedgehog pathway activation, and the present application provides a method for treating a disease caused by abnormal activation of hedgehog signaling pathway, and the present application further provides a method for treating interstitial pneumonia, the method comprising administering to a mammalian subject a therapeutically effective amount of at least one of the pharmaceutically acceptable salt of the compound of formula (I), a pharmaceutical composition according to the present application, or a combined application composition 2.

The methods of the present application include the use of at least one of the pharmaceutically acceptable salt of the compound of formula (I), which inhibits hedgehog pathway activation in regulation of various cells, tissues and organs and/or regulation of functional properties, and has the following treatment and cosmetic applications: regulation of nerve tissue, formation and repair of bone and cartilage, regulation of sperm generation, regulation of smooth muscle, regulation from original intestinal lung, liver and other organs, regulation of hematopoietic function, regulation of skin and hair growth. Thus, the methods and compounds of the present application, compositions comprising the use of inhibitors of the present application, may relate to all of these uses for inhibitors of hedgehog proteins. In addition, the methods of the present application can be carried out on cells provided in culture (in vitro) or in whole animal cells (in vivo).

The present disclosure will be described below with reference to the accompanying drawings and further detailed description. It should be noted that the following description is merely an example of the technical solutions set forth in this application, and is not any limitation on these technical solutions. The scope of protection of the present application is subject to the content of the appended claims.

The instruments and methods of detection used herein are as follows:

### X-ray Powder Diffraction (XRPD)

The XRPD pattern was collected on an X-ray powder diffraction analyzer produced by Panalytalcal or Bruker, and the scan parameters are shown in the table below.

### XRPD parameters

| Parameters | Instrument 1 | Instrument 2 | Instrument 3 |
|---|---|---|---|
| Model | Empyrean | X' Pert3 | X' Pert3 |
| | Cu, Kα, Kα1 (Å): 1.540598, | Cu, Kα, Kα1 (Å): 1.540598, | Cu, Kα, Kα1 (Å): 1.540598, |
| X ray | Kα2 (Å): 1.544426 | Kα2 (Å): 1.544426 | Kα2 (Å): 1.544426 |
| | Kα2/Kα1 intensity ratio: 0.50 | Kα2/Kα1 intensity ratio: 0.50 | Kα2/Kα1 intensity ratio: 0.50 |
| Setting of X-ray light tube | 45 kV, 40 mA | 45 kV, 40 mA | 45 kV, 40 mA |
| Divergent slit | Automatic | 1/8° | 1/8° |
| Mode of scanning | Continuous | Continuous | Continuous |
| Scan range (°2θ) | 3°-40° | 3°-40° | 3°-40° |
| Scan time per step (s) | 17.8 | 46.7 | 46.7 |
| Scan step (°2θ) | 0.0167 | 0.0263 | 0.0263 |
| Test time (s) | ~5 min 30 s | ~5 min | ~5 min |

In the table, "~" denotes "about".

### Thermogravimetric Analysis (TGA) and Differential Scanning Calorimetry (DSC)

TGA and DSC profiles were collected respectively on a TA Q500/5000/5500 thermal gravimetric analyzer and a TA Q2000/25000 differential scanning calorimeter, and the test parameters were shown in table below.

### TGA and DSC parameters

| Parameters | TGA | DSC |
|---|---|---|
| Method | Linear heating up | Linear heating up |
| Sample plate | Aluminum pan, open | Aluminum pan, cover/uncover |
| Temperature range | Room Temperature- Set the end point temperature | 2 5 ° C - Set the end point temperature |
| Scan rate (°C/ min) | 10 | 10 |
| Protective gas | Nitrogen | Nitrogen |

### mDSC parameters

| Parameters | Set value |
|---|---|
| Test Mode | Conventional mDSC |
| Amplitude | 1.0 °C |
| Modulation period | 60 s |
| Scan rate | 3.0 °C/min |
| Protective gas | N₂ at 50.00 mL/min |

### Dynamic Vapor Sorption (DVS)

The DVS curves were collected on a DVS Intrinsic from SMS (Surface Measurement Systems).The relative humidity at 25°C was corrected with the deliquescence points of LiCl, Mg (NO₃)₂ and KCl. The DVS test parameters were shown in table below.

### DVS Parameters

| Parameters | Set value |
|---|---|
| temperature | 25 °C |
| Sample amount | 10-20 mg |
| Protective gas and flow rate | Nitrogen, 200 mL/min |
| dm/dt | 0.002 %/min |
| Minimum dm/dt balance time | 10 min |
| Maximum dm/dt balance time | 180 min |
| RH range | 0 %RH-95 %RH |
| RH gradient | 10 % (0 %RH-90 %RH, 90 %RH-0 %RH) |
| | 5 % (90% RH-95 %RH, 95 %RH-90 %RH) |

### Solution NMR

The liquid nuclear magnetic hydrogen spectrum was collected on a Bruker 400M nuclear magnetic resonance instrument, and DMSO-d6 was used as a solvent.

### High Performance Liquid Chromatography (HPLC)

The purity test, the dynamic solubility and the stability test are tested by the Agilent 1260/1100 high performance liquid chromatograph, and the analysis conditions and chiral purity test analysis conditions of the high performance liquid chromatography test are respectively as follows.

### HPLC Parameters

| Instrument | Agilent 1260, DVD/VWD detector | |
|---|---|---|
| Chromatographic column | Agilent Eclipse C18, 100×4.6 mm, 3.5 µm | |
| Mobile phase | A: 0.05% TFA in H₂O | |
| | B: ACN | |
| | Time (min) | %B |
| | 0.0 | 10 |
| Gradient | 10.0 | 90 |
| | 10.5 | 90 |
| | 15.0 | 10 |
| Run time | 15.0 min | |
| flow rate | 1.0 mL/min | |
| Injection volume | 2/5/10 µL | |
| Wave length of detection | UV at 254 nm | |
| Column temperature | 25 °C | |
| Automatic injection temperature | RT | |
| Diluted solvent | ACN /H₂O=1:1 (v/v) | |

| Chiral purity test analysis conditions | | |
|---|---|---|
| Instrument | Agilent 1100 DVD detector | |
| Chromatographic column | Chiralcel OD-H, 250×4.6 mm, 5 µm | |
| Mobile phase | A: Heptane/EtOH=95:5 (0.1%DEA) | |
| Gradient | Time (min) | %A |
| | 0.0 | 100 |
| | 35.0 | 100 |
| Run time | 35.0 min | |
| flow rate | 1.0 mL/min | |
| Injection volume | 5 µL | |
| Wavelength of detection | UV at 254 nm | |
| Column temperature | 40 °C | |
| Automatic injection temperature | RT | |
| Diluted solvent | EtOH | |

The abbreviation of the solvent and the corresponding name in this specification are shown in the following table, and all solvents can be commercially available.

### Comparison table of solvent names and abbreviations used in experiments

| Abbreviation | Name | Abbreviation | Name |
|---|---|---|---|
| MeOH | Methanol | THF | Tetrahydrofuran |
| EtOH | Ethanol | Cyclohexane | Cyclohexane |
| IPA | Isopropyl alcohol | ACN | Acetonitrile |
| acetone | Acetone | DCM | Methylene chloride |
| MIBK | Methyl isobutyl ketone | toluene | Toluene |
| EtOAc | Ethyl acetate | n-heptane | n-heptane |
| IPAc | Isopropyl acetate | NMP | N-methylpyrrolidone |
| MTBE | Methyl tert-butyl ether | H₂O | Water |

### Example 1: Salt Type Screening of Compound of Formula (I)

1.1 The free base amorphous form of the compound of formula (I) was used as raw material, and six acids were selected to perform a salt-type screening test in different solvent systems. The results are shown in Table 1-1.

**Table 1-1 Salt-type Screening Test Results**

| Acid | Operation and Results |
|---|---|
| Hydrochloric Acid | The compound of formula (I) was dissolved in EtOAc, HCl gas was introduced, and there was a significant solid precipitation. |
| Tartaric acid | The compound of formula (I) was dissolved in EtOAc, DCM, toluene, tetrahydrofuran, acetone and other solvents, respectively, and tartaric acid was added, stirred and crystallized at room temperature, and no obvious solid was precipitated. |
| Camphorsulfonic acid | The compound of formula (I) was dissolved in EtOAc, DCM, toluene, tetrahydrofuran, acetone, ethanol and other solvents, respectively, camphorsulfonic acid was added, tirred and crystallized and crystallized, and no obvious solid was precipitated. |
| 1,5-Naphthalenedicarboxylic acid | The compound of formula (I) was dissolved in EtOAc, DCM, toluene, tetrahydrofuran, acetone, ethanol and other solvents, respectively, 1,5-Naphthalenedicarboxylic acid was added, stirred and crystallized at room temperature, and no obvious solid was precipitated. |
| Formic acid | The compound of formula (I) was dissolved in EtOAc, DCM, toluene, tetrahydrofuran, acetone, ethanol and other solvents, respectively, formic acid was added, stirred and crystallized at room temperature, and no obvious solid was precipitated. |
| Fumaric acid | The compound of formula (I) was dissolved in acetone, fumaric acid was added, stirred and crystallized at room temperature, a large amount of solid was separated out, after suction filtration, the filter cake had good traits and did not absorb moisture. |

1.2 The amorphous free base of the compound of formula (I) was used as raw material, and 15 acids (hydrochloric acid is used as two feed ratios) were selected to perform 48 salt-type screening tests in three solvent systems. About 15 mg of free base was stirred with equimolar acid (the molar ratio of hydrochloric acid to free base being 1: 1 and 2: 1) in 0.5 mL of solvent for 5 days, the sample was centrifuged and the solid was characterized by XRPD, and the results showed that four potential salt forms were obtained in the salt screening test (Table 1-2). The results of the XRPD pattern of succinate crystal form A are shown in FIG. 1 and Table 1-3; the results of the XRPD pattern of succinate crystal form B are shown in FIG. 2 and Table 1-4.

**Table 1-2 Salt-type Screening Test Results**

| NO. | Acid (Acid/Base Molar Ratio ) | A MTBE | B Acetone/n-heptane (1:4, v/v) | C EtOAc/Cyclohexane (1:4, v/v) |
|---|---|---|---|---|
| 1 | HCl (1:1) | Amorphous ^{b} | Amorphous ^{c} | Amorphous ^{b} |
| 2 | HCl (2:1) | Amorphous ^{b} | Amorphous ^{b} | Amorphous ^{b} |
| 3 | Sulphuric acid (1:1) | Amorphous ^{a} | Amorphous ^{b} | Amorphous ^{b} |
| 4 | Maleic acid (1:1) | Amorphous ^{b} | Amorphous ^{b} | Acid ^{b} |
| 5 | Phosphorous acid (1:1) | Amorphous ^{a} | Amorphous ^{b} | Amorphous ^{b} |
| 6 7 | Mucic acid (1:1) | Acid a | Acid ^{b} | Acid ^{a} |
| | L-tartaric acids (1:1) | Acid a | Amorphous ^{c} | Acid ^{b} |
| 8 | Fumaric acid (1:1) | Acid ° | Fumarate crystal form A + acid ^{a} | Fumarate crystal form B + acid^{a} |
| 9 | Citric acid (1:1) | Acid a | Acid ^{c} | Acid ^{b} |
| 10 | Hippuric acid (1:1) | Acid a | Acid ^{b} | Acid ^{a} |
| 11 | Succinic acid (1:1) | Succinate crystal form A+ acid ^{c} | Succinate crystal form B+ acid ^{a} | Acid ^{a} |
| 12 | Adipic acid1:1) | Acid ^{a} | Acid ^{b} | Acid ^{b} |
| 13 | p-Toluenesulfonic acid (1:1) | Amorphous ^{b} | Amorphous ^{b} | Amorphous ^{b} |
| 14 | Methanesulfonic acid (1:1) | Amorphous ^{b} | Amorphous ^{c} | Amorphous ^{b} |
| 15 | Benzenesulfonic acid (1:1) | Amorphous ^{b} | Amorphous ^{b} | Amorphous ^{b} |
| 16 | Gentisic acid (1:1) | Amorphous ^{c} | Amorphous ^{c} | Amorphous ^{b} |
| 17 | Blank | Amorphous ^{c} | Amorphous ^{c} | Amorphous ^{c} |

| | | | | |
|---|---|---|---|---|
| ^{a}: Samples were obtained by stirring at room temperature for 5 days; ^{b}: Samples were obtained by stirring at 5°C for 3 days and -20°C for 2 days; ^{c}: Samples were obtained by stirring at 5°C for 3 days and -20°C for 2 days, and then volatilizing at room temperature. | | | | |

Table 1-1, Table 1-2 salt screening test results show that although the compound of formula (I) can theoretically form salts with inorganic or organic acids, however, under various solvent conditions, compounds of formula (I) can not form crystalline salts with most acids, and Table 1-2 shows that stable crystal form of salt of compound of formula (I) can be obtained when the compound of formula (I) is reacted only with fumaric acid and succinic acid.

**Table 1-3 XRPD diffraction peak data of succinate crystal form A**

| No. | 2θ (°) | Relative intensity (%) |
|---|---|---|
| 1 | 22.06 | 100.00 |
| 2 | 27.17 | 31.38 |

**Table 1-4 XRPD diffraction peak data of succinate crystal form B**

| No. | 2θ (°) | Relative intensity (%) | No. | 2θ (°) | Relative intensity (%) |
|---|---|---|---|---|---|
| 1 | 9.54 | 55.51 | 7 | 19.18 | 28.52 |
| 2 | 12.18 | 14.05 | 8 | 22.27 | 22.69 |
| 3 | 14.37 | 86.97 | 9 | 23.65 | 55.57 |
| 4 | 15.12 | 53.48 | 10 | 24.04 | 21.76 |
| 5 | 15.62 | 26.19 | 11 | 25.54 | 32.16 |
| 6 | 16.50 | 13.92 | 12 | 27.26 | 12.11 |

### Example 2: Preparation of Fumarate Crystal Form of the Compound of Formula (I)

### 2.1 Preparation of Fumarate Crystal Form A of the Compound of Formula (I)

a. 100 mg of free base amorphous sample and 12.5 mg of fumaric acid (molar ratio of 0.5: 1, acid/base) were used as raw materials, and the mixture was suspended and stirred at room temperature in Acetone/n-Heptane (1: 4, v/v) for 18 hours in the dark, centrifuged to obtain a solid, and dried at 50°C for 24 hours.

The XRPD characterization results were shown in FIG. 3 and Table 2-1. TGA/ DSC results (a in FIG.4, b in FIG.4) show that the weight loss is 1.4% when heated to 150°C and there are two endothermic peaks at 127.3°C and 146.1°C (peak temperature). The ¹H NMR result (FIG.5) shows that the molar ratio of fumaric acid to free base in the fumarate crystal form A sample is 0.5: 1, and no solvent residue is found. In view of the small TGA weightlessness of the fumarate crystal form A and no other DSC signals observed before melting, it is determined that it is anhydrous crystal form.

**Table 2 -1 XRPD diffraction peak data of fumarate crystal form A**

| No. | 2θ (°) | Relative intensity (%) | No. | 2θ (°) | Relative intensity (%) |
|---|---|---|---|---|---|
| 1 | 6.01 | 99.96 | 10 | 17.59 | 35.64 |
| 2 | 6.49 | 52.22 | 11 | 18.14 | 26.21 |
| 3 | 9.90 | 32.32 | 12 | 19.00 | 59.15 |
| 4 | 12.21 | 46.41 | 13 | 19.57 | 35.69 |
| 5 | 12.93 | 68.16 | 14 | 20.66 | 41.58 |
| 6 | 13.27 | 62.04 | 15 | 23.28 | 25.69 |
| 7 | 13.65 | 37.63 | 16 | 25.53 | 13.20 |
| 8 | 15.43 | 49.32 | 17 | 28.26 | 18.19 |
| 9 | 16.17 | 100.00 | | | |

Other preparation examples of the fumarate crystal form A include:
b. 30 mg of amorphous free base and fumaric acid (molar ratio of 0.5: 1, acid/base) were used as raw materials, stirred at room temperature in Acetone/n-Heptane (1: 4, V/V) for 3 days, and stirred at 5°C for 3 days to obtain the crystal;
c. 1.1 g of free base and fumaric acid (the molar ratio of acid-base feeding was 0.5: 1) was suspended and stirred for 5 days in Acetone/n-Heptane (1: 4, V/V) to obtain the crystal;
d. 50 mg of the free base amorphous sample and fumaric acid (molar ratio of 0.5: 1, acid/base) were stirred at room temperature in acetone/n-Heptane (1: 4, v/v) in the dark at room temperature for 2 days, and dried under vacuum at room temperature for 18 hours to obtain the the crystal.

### 2.2 Preparation of Fumarate Crystal Form B of the Compound of Formula (I)

a. 28.7 mg of free base amorphous sample and 3.8 mg of fumaric acid (molar ratio of about 0.5: 1, acid/base) were used as raw materials, the mixture was suspended and stirred in 1mL EtOAc/Cyclohexane (1: 4, v/v) in the dark for 2 days at room temperature, after centrifugation, the product was vacuum dried at room temperature for 22 hours to obtain the fumarate crystal form B.

The XRPD characterization results are shown in FIG.6 and Table 2-2. TGA/ DSC results (a in FIG.7, b in FIG.7) show that the weight loss is 9.0% when heated to 150°C and there is one endothermic peak at 130.3°C (peak temperature). The 1H NMR result (FIG.8) shows that the molar ratio of fumaric acid to free base in the fumarate crystal form B sample is 0.6: 1, and cyclohexane solvent residues are found. The sample is subjected to variable temperature XRPD detection, and the result (FIG.9) shows that the crystal form is unchanged (the partial diffraction peak offset at 110°C is presumed to be caused by lattice expansion at high temperature) when the fumarate crystal form B is heated to 110°C under a nitrogen atmosphere and cooled to 30°C, therefore it is determined that it is anhydrous crystal form.

**Table 2-2. XRPD diffraction peak data of fumarate crystal form B**

| No. | 2θ (°) | Relative intensity (%) | No. | 2θ (°) | Relative intensity (%) |
|---|---|---|---|---|---|
| 1 | 12.22 | 15.95 | 11 | 19.06 | 33.18 |
| 2 | 12.73 | 42.46 | 12 | 20.37 | 39.05 |
| 3 | 13.19 | 52.46 | 13 | 20.93 | 14.32 |
| 4 | 15.36 | 73.84 | 14 | 22.85 | 36.88 |
| 5 | 15.78 | 90.75 | 15 | 25.87 | 19.04 |
| 6 | 16.04 | 100.00 | 16 | 26.83 | 42.19 |
| 7 | 16.56 | 64.58 | 17 | 27.96 | 27.22 |
| 8 | 17.21 | 40.05 | 18 | 28.82 | 86.12 |
| 9 | 18.03 | 41.17 | 19 | 29.42 | 34.35 |
| 10 | 18.62 | 87.07 | | | |

Other preparation examples of fumarate crystal form B include:
b. 30 mg of amorphous free base and fumaric acid (molar ratio of 0.5: 1, acid/base) were used as raw materials, stirred at room temperature in EtOAc/Cyclohexane (1: 4, V/V) for 3 days, and dried at room temperature for 2 days to obtain the crystal.

### 2.3 Preparation of Fumarate Crystal Form C of the Compound of Formula (I)

a. Approximately 15 mg of fumarate crystal Form A of the compound of formula (I) was weighed into an HPLC vial, and 0.5 mL of ACN was added. The resulting turbid solution was magnetically stirred (1000 rpm) at room temperature in the dark for 5 days, and then centrifuged (10000 rpm, 2 min) to collect the solid and cooled to dry at room temperature.

The XRPD characterization results are shown in FIG.10 and Table 2-3. TGA/DSC results (a in FIG.11, b in FIG.11) show the weight loss is 1.7% when heated to 150°C and there is one endothermic peak at 144.6°C (peak temperature). The 1H NMR result (FIG.12) shows that the molar ratio of fumaric acid to free base in the fumarate crystal form C sample is 0.5: 1, and no solvent residue is found. In view of the small TGA weightlessness of the fumarate crystal form C and no other DSC signals observed before melting, it is determined that it is anhydrous crystal form.

**Table 2-3. XRPD diffraction peak data of fumarate crystal form C**

| No. | 2θ (°) | Relative intensity (%) | No. | 2θ (°) | Relative intensity (%) |
|---|---|---|---|---|---|
| 1 | 5.03 | 24.28 | 8 | 17.80 | 45.84 |
| 2 | 10.06 | 30.69 | 9 | 19.41 | 90.07 |
| 3 | 11.04 | 44.96 | 10 | 20.22 | 21.99 |
| 4 | 12.85 | 18.17 | 11 | 21.74 | 51.09 |
| 5 | 15.11 | 100.00 | 12 | 25.15 | 7.65 |
| 6 | 16.06 | 22.69 | 13 | 27.14 | 32.69 |
| 7 | 17.13 | 54.77 | | | |

Other preparation examples of the fumarate crystal form C include:
b. 30.2 mg of amorphous free base was weighed and 1.0 mL ACN was added to dissolve to obtain an alkali solution. 3.8 mg of fumaric acid was weighed, and 0.5 mL ACN was added to dissolve the fumaric acid to obtain an acid solution. The alkali liquor was added dropwise to the acid liquor. The obtained suspension was suspended and stirred at room temperature in the dark for 10 hours, transferred to 5°C, suspended and stirred for 15 hours avoiding light, and centrifuged and separated. The solid was dried under vacuum at room temperature overnight.

### 2.4 Preparation of Fumarate Crystal Form D of the Compound of Formula (I)

a. About 15 mg of fumarate crystal form A of the compound of formula (I) was weighed into an HPLC vial, and 0.5 mL of MTBE was added to obtain a turbid solution. The solution was placed in the dark at room temperature with magnetic stirring (1000 rpm) for 6 days, and then centrifuged (10000 rpm, 2 min) to collect the solid and air-dry at room temperature.

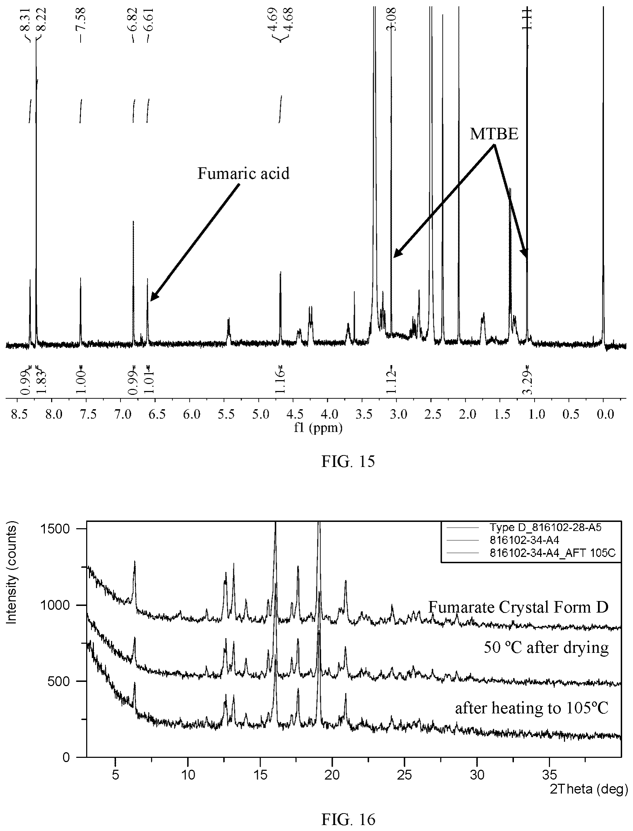

The XRPD characterization results are shown in FIG.13 and Table 2-4. TGA/ DSC results (a in FIG.14, b in FIG.14) show that weight loss is 8.9% when heated to 150°C and there is one endothermic peak at 124.7°C (peak temperature). The ¹H NMR result (FIG.15) show that the molar ratio of fumaric acid to free base in the fumarate crystal form D sample was 0.5:1, the molar ratio of residual solvent MTBE to free base is 0.4: 1, and the corresponding weight loss is 6.3%.

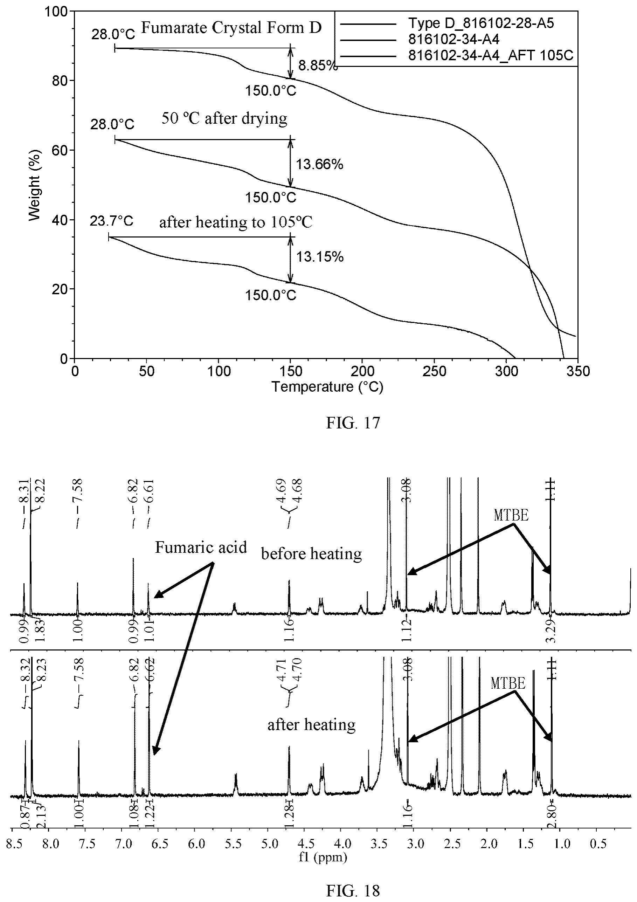

To study the TGA weight loss of fumarate crystal form D, the sample was vacuum dried and heated at 50 °C. The XRPD result (FIG.16) shows that the fumarate crystal form D does not change its crystal form after vacuum drying at 50°C for 9 hours, and the dried sample does not change its crystal form after being heated to 105°C and cooled to room temperature. The TGA result of the sample after drying and heating (FIG.17) shows that the weight loss of the sample is increased, and it is presumed that the heated sample absorbs water in the air. ¹ H NMR (FIG.18) shows that the residual solvent MTBE content remains substantially unchanged after the sample is heated.

**Table 2-4. XRPD diffraction peak data of fumarate crystal form D**

| | 2θ (°) | Relative intensity (%) | No. | 2θ (°) | Relative intensity (%) |
|---|---|---|---|---|---|
| 1 | 6.34 | 29.23 | 10 | 19.10 | 100.00 |
| 2 | 9.39 | 3.07 | 11 | 20.45 | 8.25 |
| 3 | 11.31 | 6.41 | 12 | 20.93 | 28.88 |
| No. 4 | 12.66 | 30.44 | 13 | 21.98 | 5.27 |
| 5 | 13.18 | 38.03 | 14 | 24.15 | 9.44 |
| 6 | 14.04 | 13.49 | 15 | 25.61 | 8.16 |
| 7 | 16.07 | 70.29 | 16 | 25.98 | 8.58 |
| 8 | 17.21 | 12.82 | 17 | 26.95 | 6.94 |
| 9 | 17.63 | 37.74 | 18 | 28.61 | 7.45 |

Other preparation examples of the fumarate crystal form D include:
b. About 15 mg of fumarate crystal form A of the compound of formula (I) was weighed into an HPLC vial, and 0.5 mL NMP/toluene (1: 9, v/v) was added to obtain a turbid solution, the turbid solution was placed in the dark at 5°C with magnetic stirring (1000 rpm) for 5 days, then the solid was collected by centrifugation (10000 rpm, 2 min), and dried at room temperature.

### 2.5 Preparation of Fumarate Crystal Form E of the Compound of Formula (I)

About 15 mg of fumarate crystal form A of the compound of formula (I) was weighed into an HPLC vial, and 0.5 mL IPA/n-heptane (1:4) was added, after magnetic stirring (1000 rpm) for 5 days in dark at room temperature, then the solid was collected by centrifugation and dried at room temperature.

The XRPD characterization results are shown in FIG.19 and Table 2-5. TGA/ DSC results (a in FIG.20, b in FIG.20) show that the weight loss is 5.5% at 150°C and there is one endothermic peak at 143.3°C (peak temperature). 1H NMR results (FIG.21) show that the molar ratio of fumaric acid to free base in the fumarate crystal form E sample is 0.5:1, and no residual solvent is detected.

After the sample was dried under vacuum at 50°C for 9 hours, the XRPD result (FIG.22) showed the crystal form unchanged. Since the NMR result show that the fumarate crystal form E does not contain residual solvent, TGA weight loss is large and the crystal form is unchanged after vacuum drying at 50°C, it is a hydrate or anhydrous crystal form that is hygroscopic (surface water adsorption).

**Table 2-5 XRPD diffraction peak data of fumarate crystal form E**

| No. | 2θ (°) | Relative intensity (%) | No. | 2θ (°) | Relative intensity (%) |
|---|---|---|---|---|---|
| 1 | 12.80 | 23.28 | 6 | 19.74 | 16.60 |
| 2 | 15.18 | 32.47 | 7 | 20.54 | 33.29 |
| 3 | 16.01 | 100.00 | 8 | 21.96 | 20.71 |
| 4 | 16.82 | 18.41 | 9 | 23.90 | 10.63 |
| 5 | 18.06 | 17.23 | 10 | 25.99 | 20.08 |

### 2.6 Preparation of Fumarate Crystal Form F of the Compound of Formula (I)

a. 500.4 mg of the amorphous free base and 62.6 mg of fumaric acid (molar ratio of 0.5: 1, acid/base) were suspended and stirred at room temperature in 10 mL acetone/n-heptane (1: 4, v/v) in the dark for 2 days, then centrifuged and separated, and then dried under vacuum at room temperature for 22 hours.

The XRPD characterization results are shown in FIG.23 and Table 2-6. TGA/DSC results (a in FIG.24, b in FIG.24) show that the weight loss is 2.7% when heated to 150°C and there is one endothermic peak at 158.8°C (peak temperature). The ¹H NMR result (FIG.25) shows that the molar ratio of fumaric acid to free base in fumarate crystal form F sample is 0.5:1, and no residual solvent is detected. The sample was subjected to variable temperature XRPD detection, and the result (FIG.26) showed that the crystal form was unchanged (the partial diffraction peak offset at 140°C was presumed to be caused by lattice expansion at high temperature) when the fumarate crystal form F was heated to 140°C under nitrogen atmosphere and cooled to 30°C, and therefore it was determined that it was anhydrous crystal form.

**Table 2-6. XRPD diffraction peak data of fumarate crystal form F**

| | 20 (°) | Relative intensity (%) | No. | 2θ (°) | Relative intensity (%) |
|---|---|---|---|---|---|
| 1 | 5.54 | 18.41 | 11 | 20.95 | 32.70 |
| 2 | 9.57 | 4.06 | 12 | 21.49 | 14.24 |
| 3 | 11.06 | 100.00 | 13 | 22.38 | 38.21 |
| 4 | 14.46 | 15.47 | 14 | 23.31 | 9.78 |
| No. 5 | 15.47 | 14.44 | 15 | 25.03 | 25.41 |
| 6 | 16.60 | 55.55 | 16 | 27.44 | 7.39 |
| 7 | 16.90 | 28.06 | 17 | 28.86 | 8.10 |
| 8 | 17.49 | 15.01 | 18 | 29.89 | 7.64 |
| 9 | 18.64 | 33.33 | 19 | 30.45 | 7.92 |
| 10 | 19.16 | 6.43 | | | |

Other preparation examples of the fumarate crystal form F include:
b. Obtained by suspension stirring of fumarate crystal form A of the compound (I)

About 15 mg of fumarate crystal form A of the compound of formula (I) was weighed to HPLC glass vial, and 0.5 mL of the solvent listed in Table 2-7 was added respectively. The turbid liquid obtained was placed under magnetic stirring (1000 rpm) at different temperatures for about 5 days, then centrifuged (10000 rpm, 2 min) to collect the solid and perform XRPD testing. The test results are shown in Table 2-7.

**Table 2-7 Results of Room Temperature Suspension Stirring Test**

| NO. | Solvent | Temperature | Time | Result |
|---|---|---|---|---|
| 1 | MTBE | 50°C | 5d | form F |
| 2 | MIBK | 5°C | 5d | form F |

### c. Obtained by the compound of formula (I) reacting with fumaric acid

The compounds of formula (I) shown in Table 2-8, along with the corresponding proportions of fumaric acid, were suspended and stirred at room temperature in the indicated solvents in the dark for 4 days, centrifuged, and then dried under vacuum at room temperature to prepare the product. The test results are shown in Table 2-8.

**Table 2-8. Preparation of Fumarate Crystal Form F of the Compound of Formula (I)**

| Material | Amount (mg) | ratio | Solvent(v/v) | Operation | Time | Result |
|---|---|---|---|---|---|---|
| Free base +acid | 10 | 0.5:1 | ACN | room temperature, Light Shielding, Suspension Stirring | 4d | form F |
| Free base +acid | 50 | 0.5:1 | MTBE | room temperature, Light Shielding, Suspension Stirring | 4d | form F |
| Free base +acid | 50 | 0.5:1 | IPA/n-heptane (1:4) | room temperature, Light Shielding, Suspension | 4d | form F |
| | | | | Stirring | | |
| Free base +acid | 50 | 0.5:1 | EtOH/H₂O (1:9) | 50°C ,Light Shielding , Suspension Stirring | 4d | form F |
| Free base +acid | 50 | 0.5:1 | IPA/n-heptane (1:4) | 50°C ,Light Shielding , Suspension Stirring | 4d | form F |

### 2.7 Preparation of Fumarate Crystal Form G of the Compound of Formula (I)

a. 15 mg of fumarate crystal form A of the compound of formula (I) was weighed into an HPLC glass vial, 0.5 mL EtOH/H₂O (1:9) was added, and the obtained suspension was magnetic stirred (1000 rpm) at 50°C in the dark for 5 days, and then centrifuged (10000 rpm, 2 min) to collect the solid.

The XRPD characterization results are shown in FIG.27 and Table 2-9. TGA/DSC results (a in FIG. 28, b in FIG. 28) show that the weight loss is 4.0% when heated to 150°C and there are two endothermic peaks at 70.8°C and 170.1°C (peak temperature). The ¹H NMR result (FIG.29) shows that the molar ratio of fumaric acid to free base in the fumarate crystal form G sample is 0.3:1, and the molar ratio of the residual solvent EtOH to the free base is 0.3: 1.

**Table 2-9. XRPD diffraction peak data of fumarate crystal form G**

| No. | 20 (°) | Relative intensity (%) |
|---|---|---|
| 1 | 15.60 | 66.35 |
| 2 | 17.69 | 100.00 |
| 3 | 20.63 | 33.24 |

Other preparation examples of the fumarate crystal form G include:
b. About 15 mg of the fumarate crystal form A of the compound of formula (I) was weighed into an HPLC glass vial, and 0.5 mL NMP/H₂O (1: 9) was added, and the obtained suspension was magnetic stirred (1000 rpm) at 50°C for 5 days, and then centrifuged (10000 rpm, 2 min) to collect the solid.

### 2.8 Preparation of Fumarate Crystal Form H of the Compound of Formula (I)

About 15 mg of fumarate crystal form A of the compound of formula (I) was weighed into an HPLC glass vial and 0.5 mL IPA/n-Heptane (1: 9, v/v) was added, and the obtained suspension was magnetic stirred (1000 rpm) at 50°C in the dark for 5 days, and then centrifuged (10000 rpm, 2 min) to collect the solid.

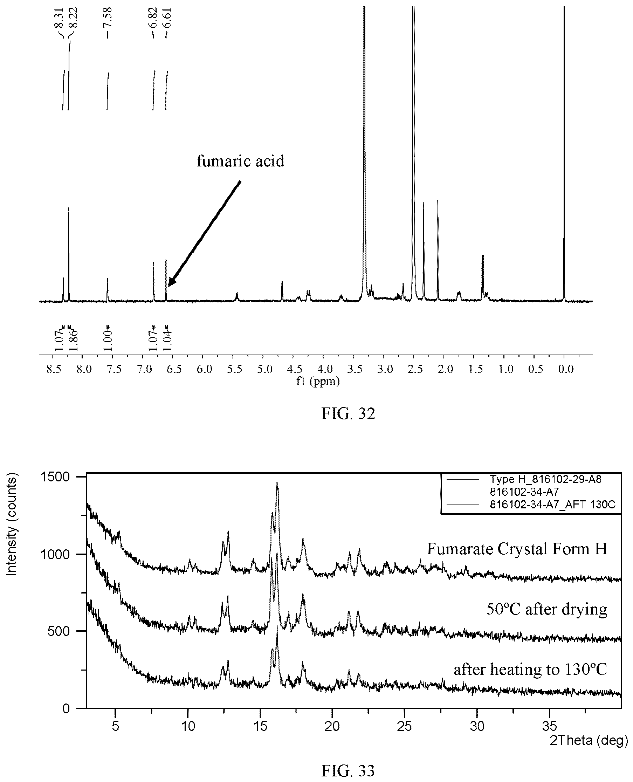

The XRPD characterization results are shown in FIG.30 and Table 2-10. TGA/DSC results (a in FIG.31, b in FIG.31) show that the weight loss is 4.4% at 150°C and there is one endothermic peak at 156.0°C (peak temperature). ¹H NMR results (FIG.32) show that the molar ratio of fumaric acid to free base in fumarate crystal form H sample is 0.5:1, and no residual solvent is detected.

**Table 2-10 XRPD diffraction peak data of fumarate crystal form H**

| No. | 2θ (°) | Rel. Int. [%] | No. | 2θ (°) | Rel. Int. [%] |
|---|---|---|---|---|---|
| 1 | 5.23 | 11.30 | 9 | 20.33 | 10.61 |
| 2 | 12.44 | 33.48 | 10 | 21.19 | 23.93 |
| 3 | 12.81 | 45.21 | 11 | 21.84 | 25.56 |
| 4 | 14.54 | 13.97 | 12 | 23.72 | 12.36 |
| 5 | 15.82 | 64.04 | 13 | 26.12 | 13.59 |
| 6 | 16.18 | 100.00 | 14 | 27.39 | 8.21 |
| 7 | 16.93 | 16.95 | 15 | 29.25 | 9.47 |
| 8 | 17.99 | 36.65 | | | |

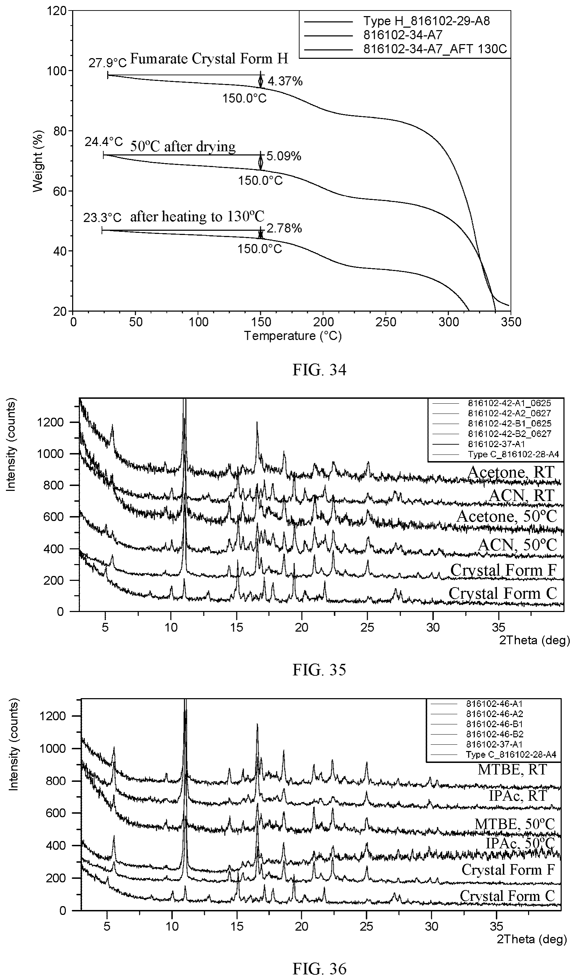

To study TGA weight of fumarate crystal form H, the sample was vacuum dried and heated at 50°C. The XRPD results (FIG.33) show that the crystal form of fumarate crystal form H remains unchanged after vacuum drying at 50°C for 9 hours, and the crystal form of dried sample remains unchanged after heating to 130°C and cooling to room temperature. The TGA result of the sample after drying and heating (FIG.34) shows that the weight loss decreases after heating. Since the NMR result shows there isn't residual solvent in fumarate crystal form H, and TGA weightlessness is large and the crystal form remain unchanged after heating, the fumarate crystal form H is hydrate or anhydrous crystal form that is hygroscopic (surface water adsorption).

### Example 3: Research on Thermodynamics Stability Relationship of Crystal Forms

To study the thermodynamic stability relationship among the crystal forms of the fumarate salt, a suspension competition experiment at room temperature and 50°C is performed on the fumarate salt anhydrous crystal forms A, B, C and F.

The fumarate crystal form F was weighed into HPLC vials, and corresponding solvent was added, then suspended and stirred at room temperature/50°C for 4 or 1.5 hours, then filtered into HPLC vials containing each crystal form by a 0.45 µm pore size PTFE filter membrane and then suspended and stirred at room temperature/50°C, respectively. XRPD was tested on each sample, and the results were summarized in Table 3-1, and the XRPD results were listed in FIG.35 and FIG.36. The crystal form F was obtained at two temperatures of each solvent, and thus the crystal form F was determined to be the most thermodynamically stable anhydrous crystal form among Forms A, B, C and F from room temperature to 50°C.

**Table 3-1 Summary of Suspension Contention Tests**

| Start Form | solvent | Temperature | Time | Solid Form |
|---|---|---|---|---|
| fumarate crystal form A+B+F | Acetone | Room temperature | 4 d | form F |
| | ACN | Room temperature | 6 d | form F+C* |
| | Acetone | 50°C | 4 d | form F |
| | ACN | 50 °C | 6 d | form F+C* |
| fumarate crystal form F+C | MTBE | Room temperature | 17h | form F |
| | IPAc | Room temperature | 17h | form F |
| | MTBE | 50°C | 17h | form F |
| | IPAc | 50°C | 17h | form F |

| | | | | |
|---|---|---|---|---|
| *: It is speculated that due to the influence of the solvent (crystal form C was obtained by solution crystallization in CAN), and the sample did not completely transform into crystal form F after stirring for 6 days. | | | | |

The above process can also be used to prepare the fumarate crystal form F from the fumarate crystal forms A, B and C.

### Example 4: Salt Type Evaluation

The fumarate crystal form F of the compound of formula (I), which is more thermodynamically stable, and the initiator amorphous free base are used for salt type evaluation, including hygroscopicity, dynamic solubility, and solid state stability evaluation.

### 4.1 hygroscopicity evaluation

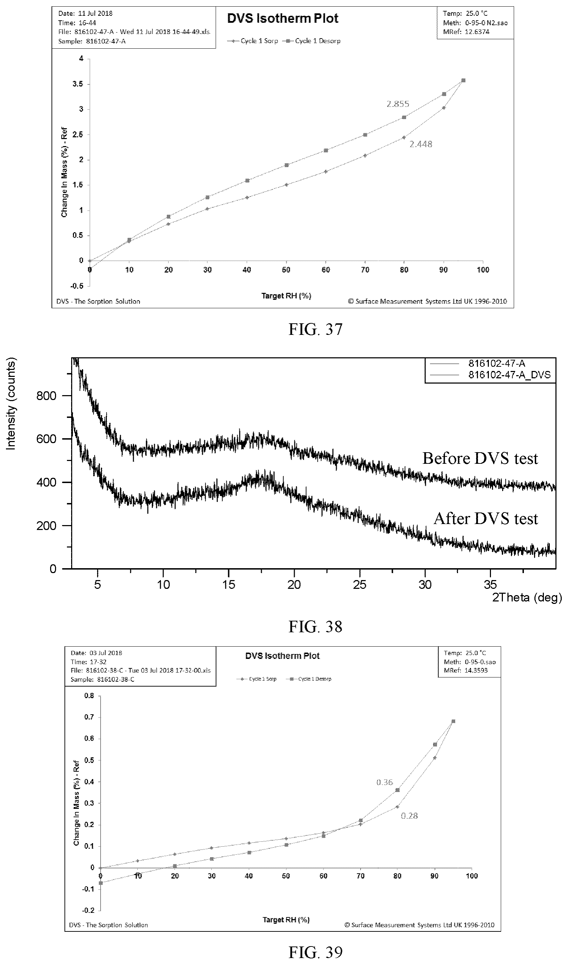

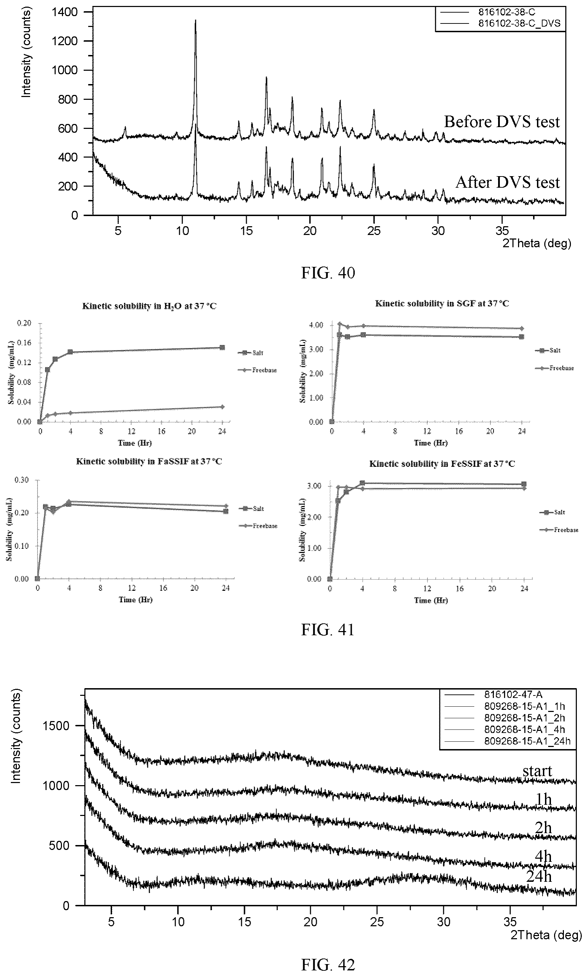

The amorphous free base of the compound of formula (I) and fumarate crystal form F of the compound of formula (I) were subjected to a hygroscopicity evaluation by a dynamic moisture adsorption (DVS) instrument. Starting with 0 humidity (0% RH), the mass change percentage of the sample was tested with humidity change (0% RH-95% RH-0% RH) at a constant temperature of 25°C.The test results are shown in FIG.37 and FIG.39, the moisture adsorption of the amorphous free base at 25°C/80% RH is 2.4%, the moisture adsorption of the fumarate crystal form F of the compound of formula (I) at 25°C/80% RH is 0.3%, and the XRPD results (FIG.38 and FIG.40) showed that no crystal transformation or crystal form change occured after the DVS test.

### 4.2 Dynamic Solubility

The dynamic solubility of amorphous free base of the compound of formula (I) and fumarate crystal form F of the compound of formula (I) were evaluated in water and three biological solvents.

### a. Preparation for Biological Solvent

### Preparation of Simulated Gastric Fluid (SGF)

0.2 g of sodium chloride and 0.1 g of Triton X -100 were weighed into a 100 mL volumetric flask, purified water was added for dissolution, stirred until the solid was completely dissolved, about 135 µL of concentrated hydrochloric acid (37%, 12 M) was added, and then the pH was adjusted to 1.8 with 1M hydrochloric acid or 1M sodium hydroxide. Finally, the volume is fixed with purified water.

### Preparation of Fasted State Simulated Intestinal Fluid (FaSSIF)

0.17 g of sodium dihydrogen phosphate (NaH2PO4, anhydrous), 0.021 g of sodium hydroxide and 0.31 g of sodium chloride were weighed into a 50 mL volumetric flask respectively, about 48 mL of purified water was added for dissolution, the pH was adjusted to exactly 6.5 with 1M hydrochloric acid or 1M sodium hydroxide, and the volume was fixed with purified water. 0.11 g of SIF powder was then added, stirred and sonicated to completely dissolve the powder. The solution can be used only after being placed at room temperature for two hours to reach equilibrium.

### Preparation for Fed State Simulated Intestinal Fluid (FeSSIF)

0.41 mL of glacial acetic acid, 0.20 g of sodium hydroxide and 0.59 g of sodium chloride were weighed into a 50 mL volumetric flask respectively, and about 48 mL of purified water was added for dissolution, the pH was adjusted to exactly 5.0 with 1M hydrochloric acid or 1M sodium hydroxide, and the volume was fixed with purified water. 0.56 g of SIF powder was then added, stirred and sonicated to completely dissolve the powder. The solution can be used only after being placed at room temperature for two hours to reach equilibrium.

### b. Solubility Test

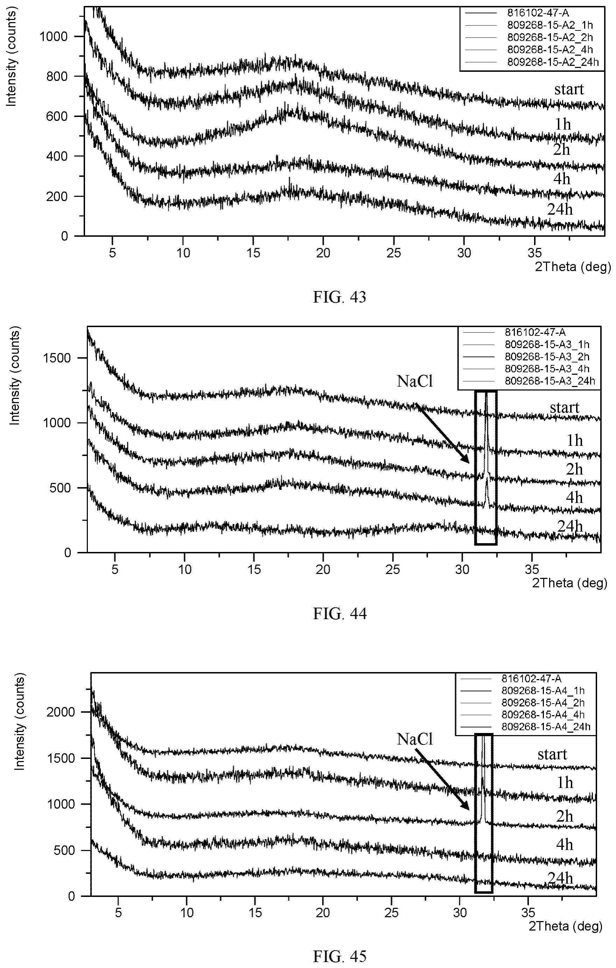

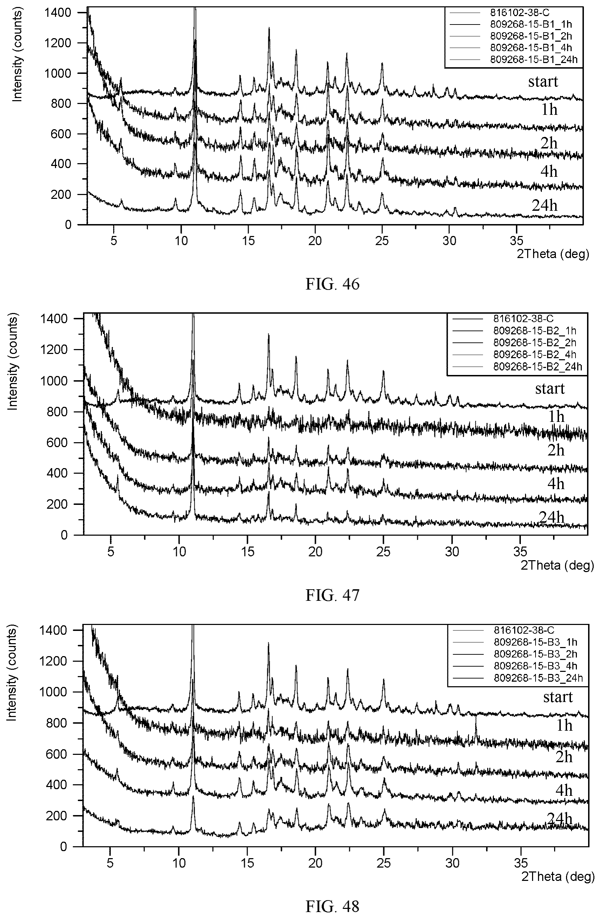

The solubility of each sample in four solvent systems of water, SGF, FASSIF, and FeSSIF (1, 2, 4, and 24 hours) was measured at a feed concentration of 5 mg/ml at 37°C using a rotary mixing (25 rpm). The sample at each time point was centrifuged and filtered (12000 rpm, 2 min, 0.45 µm PTFE filter head) to determine its HPLC solubility and pH value, and the centrifuged solid sample was tested for XRPD. The results of the solubility test are summarized in Table 4-1, and the solubility curve shows in FIG.41. The solubility results of the two samples in SGF, FASSIT and FeSSIF are similar. The XRPD results of the solubility sample are listed in FIG.42 to FIG.49, and the results show that no crystal transformation occurrs in any of the samples.

**Table 4-1 Summary of the dynamic solubility test results**

| Material | Solvent | 1 h | | 2h | | 4h | | 24 h | |
|---|---|---|---|---|---|---|---|---|---|
| | | S | Crystal variation | S | Crystal variation | S | Crystal variation | S | Crystal variation |
| Amorphous free base of the compound of formula (I) | H₂O | 0.013 | NO | 0.016 | NO | 0.018 | NO | 0.030 | NO |
| | SGF | 4.1 | NO | 3.9 | NO | 4.0 | NO | 3.9 | NO |
| | FaSSIF | 0.21 | NO | 0.20 | NO | 0.24 | NO | 0.22 | NO |
| | FeSSIF | 3.0 | NO | 3.0 | NO | 2.9 | NO | 2.9 | NO |
| Fumarate crystal form F of the compound of formula (I) | H₂O | 0.11 | NO | 0.13 | NO | 0.14 | NO | 0.15 | NO |
| | SGF | 3.6 | NO | 3.5 | NO | 3.6 | NO | 3.5 | NO |
| | FaSSIF | 0.22 | NO | 0.21 | NO | 0.23 | NO | 0.21 | NO |
| | FeSSIF | 2.5 | NO | 2.8 | NO | 3.1 | NO | 3.1 | NO |

| | | | | | | | | | |
|---|---|---|---|---|---|---|---|---|---|
| S: solubility (mg/mL) | | | | | | | | | |

### 4.4 Solid State Stability

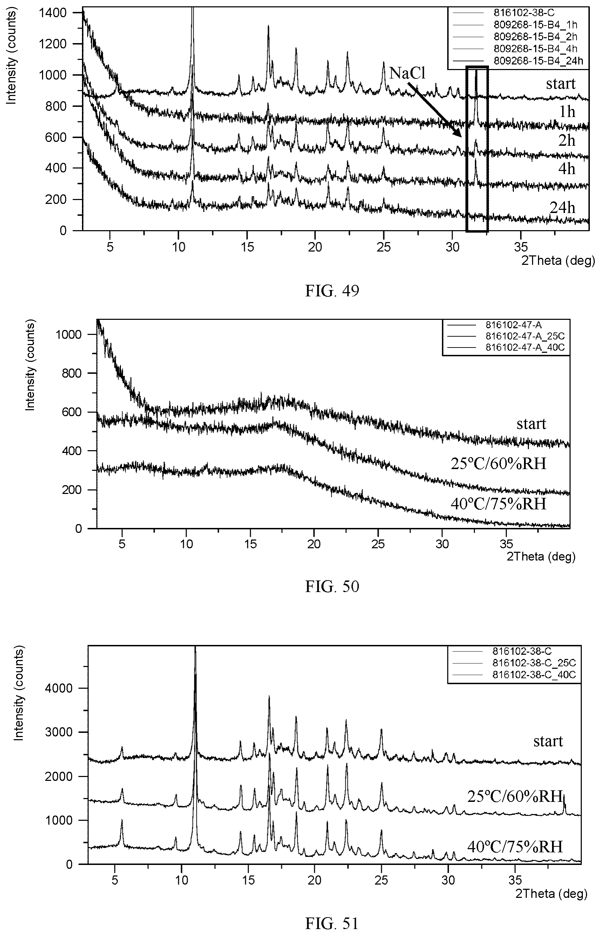

After placing the amorphous free base and fumarate crystal form F of the compound of formula (I) at 25°C/60% RH and 40°C/75% RH for 1 week, the physical and chemical stability of the sample is detected by XRPD and HPLC (at the same time the chiral purity of the sample is tested). The results of the test data are listed in Table 4-2. The results of XRPD are shown in FIG.50 and FIG.51, and the results show that two samples do not degrade or crystal transfer after being placed for 1 week at 25°C/60% RH and 40°C/75% RH, and the physical and chemical stability is good.

**Table 4-2 Solid Stability Evaluation Summary**

| Initial form | Condition. | Crystal variation | Purity (Area%) | Purity/Initial purity (%) | Chiral purity (%) |
|---|---|---|---|---|---|
| The amorphous free base form | Start | - | 99.0 | - | 98.5 |
| | 25°C/60%RH_1w | NO | 99.0 | 100.0 | 98.7 |
| | 40°C/75%RH_1w | NO | 98.9 | 99.9 | 98.7 |
| Fumarate crystal form F | Start | - | 99.6 | - | 98.6 |
| | 25°C/60%RH_1w | NO | 99.7 | 100.1 | 98.6 |
| | 40°C/75%RH_1w | NO | 99.6 | 100.0 | 98.6 |

The fumarate crystal form evaluation results of the compound of formula (I) are summarized in Table 4-3 below:

**Table 4-3 Summary of evaluation of salts of the compound of formula (I)**

| Salt form | Amorphous free base | Fumarate crystal form F |
|---|---|---|
| TGA weightlessness (150 °C) | 3.5 | 3.2 |
| DSC endothermic peak (peak temperature) | 75.9* | 161.2 |
| Water adsorption (25°C/80%RH) | 2.4 | 0.3 |
| Form change after the DVS test | NO | NO |
| Dynamic Solubility Solid State Stability | similar solubility, no crystal transformation good physical and chemical stability | |
| Whether to recommend | NO | Yes |

| | | |
|---|---|---|
| *: Glass Transition Temperature. | | |

### Example 5: Pharmacokinetics study

### 5.1 Pharmacokinetic Study in Rats

### 5.1.1 Preparation of drug Formulation:

Solvent A: 0.5% CMC-Na +0.2% Tween80

Preparation method: 5 g of sodium carboxymethyl cellulose (CMC-Na) was accurately weighed, 2 mL of Tween 80 was added, 998 mL of purified water was added, stirred and evenly mixed on a magnetic stirrer, and stored at room temperature.

Drug concentration of fumarate salt form F of the compound of formula (I): 1 mg/mL.

Preparation method: About 27 mg of the fumarate salt form F of the compound of formula I (equivalent to about 24 mg of free base) was weighed, and 24 mL of solvent A was added, then sonicated and stirred until uniform.

### 5.1.2 Animal Administration and Blood Sampling and Plasma Analysis

Male Sprague-Dawley rats (weight: 179g-290g) were purchased from Beijing Vital River Laboratory Animal Technology Co., Ltd. The oral dose is 10 mg/kg. After administration, blood was taken at different time points through orbit, and the blood samples were placed in a tube containing heparin sodium (as an anticoagulant), centrifuged by a cryocentrifuge and then stored in an environment of -80°C. An appropriate amount of plasma sample (50 µL) was taken into which (200 µL) cold acetonitrile containing the internal standard was added. The plasma protein was precipitated by centrifugation for 10 minutes. Finally, 100 µL of the supernatant was added and diluted with 100 µL of pure water and then injected into an LC-MS/MS system for analysis.

### 5.1.3 Analysis Conditions

LC-MS/MS analysis method: All samples were analyzed by an LC-MS/MS system of an API 3000 mass spectrometer equipped with an Agilent 1100 series of liquid phase systems (Agilent, Japan). Column: Agilent XORBAX, XDB-C8 (50×2.1mm;3.5µm). Column temperature: room temperature, flow rate: 0.4 mL/min, total running time:7min.

For the quantitative analysis of MS/MS, the API3000 mass spectrometer is operated in the positive ESI mode with multiple reaction monitoring (MRM). All compounds and internal standards were monitored with a dwell time of 200 milliseconds. Other MS/MS parameter settings are as follows: Nebulizer gas (NEB): 12 psi, Gas curtain gas (CUR): 7 psi, Collision gas (CAD): 4 psi, Ion spray voltage: 5000V, Ion source temperature:500 °C. For the transitions of the selected ions, each analyte was tested under the optimal sensitivity clustering potential (DP) and collision energy (CE). Finally, the data were collected and processed using the SCIEX Analyst 1.5.2 data processing software system.

### 5.1.4 Analysis of Experimental Results

The experimental results are shown in Table 5-1 (the third column), and are compared with the amorphous form of the free base of the compound in Equation (I) (the experimental data are derived from Patent WO2018006756A1, Example 5, i.e. B1):

**Table 5-1 Pharmacokinetic of the free base form and the fumarate salt form F of formula (I)**

| Compound | compound of formula (I) | the fumarate salt form F of formula (I) |
|---|---|---|
| Route of Administration | P.O. | P.O. |
| Dose (mg/kg) | 10 | 10 |
| AUC₀₋₂₄ₕ (ng*h/mL) | 12324 | 14297 |
| Cₘₐₓ (ng/mL) | 1520 | 2852 |
| Tₘₐₓ (h) | 1 | 1.10 |
| F% | 100 | 97.51 |

The results show that in the pharmacokinetics test of rats, in the same dose, compared with the free base compound B1, the Cₘₐₓ of fumarate salt form F of the compound of formula (I) is nearly doubled after oral administration in rats than that of the free base compound B1, and the drug exposure AUC of fumarate salt form F is also improved to a certain extent than that of the free base type (I) compound (B1) ,indicating that the fumarate salt form F of the compound of formula (I) improves the degree of absorption of the drug *in vivo* relative to the compound free base of formula (I).

Re-verification: The applicant uses the same formulation system to verify the fumarate salt form F and free base of the compound of formula (I) in the same system to obtain a consistent conclusion; and the Cₘₐₓ (1277 ng/mL) of the fumarate salt form F of the compound of formula (I) is significantly improved compared with the Cₘₐₓ (922 ng/mL) of the free base compound B1 after the rat is orally administered.

The preparation method of drug is as follows:
Solvent C:2.4% DMSO + 0.1% Tween 80 + 97.5% CMC-Na (0.5%)

Preparation method: 0.1 g of sodium carboxymethyl cellulose (CMC-Na) was accurately weighed, 20 ml of purified water was added, and ultrasonic stirring was performed to dissolve it to obtain 0.5% CMC-Na.

Drug concentration of fumarate salt form F of the compound formula (I): 1mg/mL.

The preparation method: weighing about 11.25 mg of fumarate salt form F of the compound formula I (converted to a free base amount of about 10 mg), adding 240 uL DMSO, and ultrasonically dissolving; and then adding 10 uL of Tween 80, then adding 9750 uL of the above 0.5% CMC-Na solution, and performing ultrasound for 3 min.

### 5.2 Pharmacokinetic Study in Mice

### 5.2.1 Preparation of drug:

The preparation of solvent A and the fumarate salt form F of the compound formula (I) is referred to 5.1.1

GDC-0449: also known as Vismodegib, available from Shanghai Top Chemical Technology Co., Ltd.;

### Concentration of GDC-0449: 1mg/mL

Preparation method of GDC-0449: 24 mg GDC-0449 was weighted, 24 mL of solvent A was added, then ultrasonic and uniform stirring.

### 5.2.2 Animal Administration and Blood Sampling

C57 mice, male; randomly divided into 2 groups and 4 animals/group, after fasting overnight, the first group of single intragastric administration gave GDC-0449, the second group of single intragastric administration gave the fumarate salt form F of the compound of formula (I), both of the administration dosage is 10 mg/kg, and both of the administration volume is 10 mL/kg. After administration, blood of the test mice was taken at different time points (0.25, 0.5, 1, 2, 4, 8, 12, 24h) through the periorbital venous plexus, the blood sample was placed in a test tube containing heparin sodium (as an anticoagulant), then the blood sample was centrifuged at 4°C and 4000 rpm for 10 min, then the upper layer plasma was taken, and the plasma was stored in an environment of -70°C for analysis.

### 5.2.3 Plasma Sample Processing and Analysis

The collected plasma samples were subjected to a protein precipitation method by using an organic reagent according to the following method to extract test article in the plasma, and the extracted sample was subjected to quantitative concentration analysis through the LC-MS/MS.

GDC-0449 Group: 50 µL of plasma at each time point was taken, 200 µL of acetonitrile was added, vortex mixing and centrifugation was performed at 10000 rpm for 10 min, and 200 µL of supernatant was taken to perform LC-MS/MS analysis. Meanwhile, an accompanying standard curve and quality control samples were prepared for quantitative analysis.

The fumarate salt form F of the compound of formula (I) Group: 50 µL of plasma at each time point was taken, 400 µL of acetonitrile was added, vortex mixing and centrifugation was performed at 10000 rpm for 10 min, and a 200 µL supernatant for LC-MS/MS analysis was taken. Meanwhile, an accompanying standard curve and quality control samples were prepared for quantitative analysis. The LC-MS/MS analysis conditions are as follows:

**Table 5-2 HPLC Analysis Conditions**

| | | |
|---|---|---|
| HPLC: | Aglient 1260 | |
| Column: | Agilent Eclipse XDB- C18 (2.1 mm*150 mm, 5µm) | |
| Mobile phase A: | 0.1% formic acid in water | |
| Mobile phase B: | acetonitrile | |
| Flow rate: | 0.4 mL/min | |
| Injection volume: | The fumarate salt form F of the compound of formula (I) | |
| | Group: 5 µL | |
| | GDC-0449 group:10 µL | |
| Run time: | 7 min | |
| Gradient: | Time (min) | B% |
| | 0.0 | 20 |
| | 1.0 | 95 |
| | 4.0 | 95 |
| | 4.5 | 20 |
| | 7.0 | 20 |

MS Conditions:
MS Instrument: API4000 QTRAP LC-MS/MS
Ion Source: ESI, Positive ion scan mode
Scan Mode: MRM
Source Parameters:

**Table 5-3 Source Parameters**

| Ion source | CUR | CAD | IS | TEM | Gas1 | Gas2 | CXP | EP |
|---|---|---|---|---|---|---|---|---|
| ESI | 20 | Medium | 5000 | 500 | 20 | 30 | 15 | 10 |

Drug parameters:

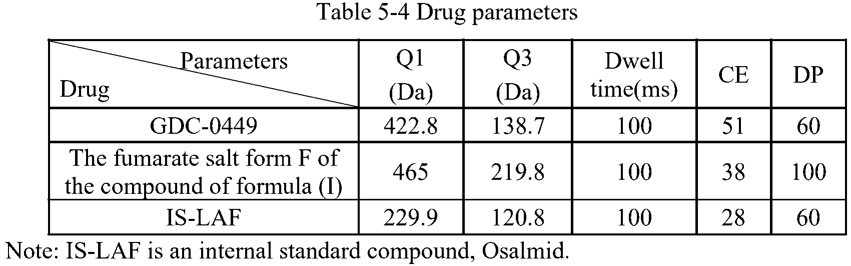

### 5.2.4 Data Analysis

According to the plasma drug concentration data obtained through analysis, the main pharmacokinetic parameters were calculated according to the non-atrioventricular model by using Kinetic software (Version 5.0).

### 5.2.5 Results

The main results of the average pharmacokinetic parameters after oral administration of 10 mg/kg dose of GDC-0449 and the fumarate salt form F of formula (I) to male mice are shown in the following table.

**Table 5-5 Pharmacokinetic parameter of GDC-0449 and the fumarate salt form F of formula (I)**

| Compound | GDC-0449 | the fumarate salt form F of formula (I) |
|---|---|---|
| Route of Administration | intragastric administration | intragastric administration |
| Dose (mg/kg) | 10 | 10 |
| Cₘₐₓ (ng/mL) | 1270 | 2413 |
| AUCₗₐₛₜ (ng·h/mL) | 6045 | 7503 |
| AUC_{inf} (ng·h/mL) | 6648 | 16717 |
| Tₘₐₓ (h) | 0.63 | 0.25 |
| T_{1/2}(h) | 8.43 | 12.37 |
| MRT(h) | 8.33 | 15.55 |

The above results show that under the same dose of intragastric administration, the Cₘₐₓ of the fumarate salt form F of the compound of formula (I) is about 1 fold higher than that of the positive drug GDC-0449 in mice, the exposure amount AUC of the fumarate salt form F of the compound of formula (I) is also higher than that of GDC-0449, and the elimination half-life T_{1/2} and the *in vivo* retention time MRT are prolonged by 4 -7 h than that of GDC-0449. So that the fumarate salt form F of the compound of formula (I) has better pharmacokinetic characteristics in mice than GDC-0449.

### Example 6: Evaluation of Antitumor Efficacy Against Medulloblastoma

### 6.1 Experimental Materials

### Compound A: fumarate salt crystal form F of the compound of formula (I);

The preparation of the administration solution is shown in Table 6-1.

**Table 6-1 Method of Drug Preparation**

| Test drug | Preparation Method | Concentration (mg/mL) |
|---|---|---|
| Vehicle | 1. Weigh 2000 mg of CMC-Na (carboxymethyl cellulose sodium). Add 400 mL of purified water and ultrasonicate to dissolve it and obtain a 0.5% CMC-Na solution. | - |
| | 2. Add 800 µL of Tween 80 and mix uniformly by ultrasonication or vortex mixing. | |
| GDC-0449 | 1. Weigh 24 mg of GDC-0449. | 1 |
| | 2. Add 24 mL of the vehicle and mix uniformly by ultrasonication and stirring. | |
| Compound A-10 mg/kg | 1. Weigh 27 mg of Compound A (calculated as free base: 24 mg). | 1 |
| | 2. Add 24 mL of the vehicle and mix uniformly by ultrasonication and stirring. | |
| Compound A-3 mg/kg | 1. Weigh 8.1 mg of Compound A (calculated as free base: 7.2 mg). | 0.3 |
| | 2. Add 24 mL of the vehicle and mix uniformly by | |
| | ultrasonication and stirring. | |
| Compound A-1 mg/kg | 1. Weigh 2.7 mg of Compound A (calculated as free base: 2.4 mg). | 0.1 |
| | 2. Add 24 mL of the vehicle and mix uniformly by ultrasonication and stirring. | |

### 6.2 Experimental Animals

60 male CB-17/SCID mice (aged 6-8 weeks) were purchased from Shanghai SLAC Laboratory Animal Co., Ltd. Animals were housed in individually ventilated cages (IVCs), with five mice per cage. Housing conditions were as follows: temperature was maintained at 20-26°C; humidity was 30-70%; 12 hours of light and 12 hours of darkness.

### 6.3 Experimental Groups

CB-17/SCID mice were subcutaneously injected with primary extracted medulloblastoma cells. After 3-4 weeks, a subset of mice developed tumors with volumes ranging from 150 to 300 mm³.

30 mice with uniform tumor sizes (Coefficient of Variation, CV < 30%, CV = Standard Deviation / Mean) were selected from this group. These mice were selected and randomly divided into five experimental groups according to Table 6-2, with six mice in each group and one group per cage. Drug administration began the day after grouping and lasted for 17 days with a frequency of twice daily (BID). The drug was discontinued after 17 days to observe tumor changes. The grouping and drug administration plan are shown in Table 6-2.

**Table 6-2 The Grouping and Drug Administration Plan**

| Group | Number of Mice | Test compound | Administration Dosage (mg/kg) | Dosage Volume | Route of Administration | Dosage Regimen |
|---|---|---|---|---|---|---|
| 1 | 6 | Vehicle | 0 | 10 mL | i.g. | BID × 17 days, followed by observation |
| 2 | 6 | GDC-0449 | 10 | 10 mL | i.g. | BID × 17 days, followed by observation |
| 3 | 6 | Compound A(10 mg/kg) | 10 | 10 mL | i.g. | BID × 17 days, followed by observation |
| 4 | 6 | Compound A (3 mg/kg) | 3 | 10 mL | i.g. | BID × 17 days, followed by observation |
| 5 | 6 | Compound A (1 mg/kg) | 1 | 10 mL | i.g. | BID × 17 days, |

| | | | | | | |
|---|---|---|---|---|---|---|
| Note: 1. Administration dosage was adjusted according to body weight (the administration volume is 10 mL/kg). | | | | | | |

### 6.4 Animal Status Observation

During routine examinations, animals were monitored for tumor growth, activity level, food intake, water consumption, weight gain or loss, eye, fur, and any other abnormal behaviors. Animal deaths and clinical abnormalities were recorded. Tumor volume (TV) was measured twice weekly using calipers and calculated using the formula: TV = 0.5a × b², wherein a is the longest diameter of the tumor and b is the shortest diameter. Body weight was recorded twice weekly, concurrently with tumor volume measurements.

Treatment was discontinued when the animals' weight decreases by 15% compared to the initial administration or when the animals' condition deteriorates significantly. If the animals' weight subsequently recovered to within a 10% loss compared to baseline, whether to continue the administration was based on the animals' condition. The in vivo experimental for an individual animal or the entire group would be terminated under the following conditions. The animals will be euthanized before death or coma.
➢ If the animals' health condition deteriorated continuously, causing persistent suffering and is unable to eat or drink.
➢ If the animal became emaciated, with a body weight loss exceeding 20% compared to the initial administration.
➢ If the tumor volume of an individual mouse exceeded 3000 mm³ or the mean tumor volume of the entire group exceeded 2000 mm³.

### 6.5 Data Results

The Student's t-test was used to compare the differences in the relative tumor volume of tumor-bearing mice at different time points between each group of the test compound and the GDC-0449 treatment group. All data were analyzed using GraphPad Prism 5.0. P value < 0.05 was considered statistically significant.

During the experiment, in Group 1 (vehicle control), one tumor-bearing mouse developed a tumor volume exceeding 3000 mm³ on day 12, and the mean tumor volume of the entire group exceeded 2000 mm³. This group's experiment was terminated, and the animals were euthanized. The remaining four groups were stopped from treatment after 17 days of administration and observed for a total of 21 days.

### 6.5.1 Body Weight

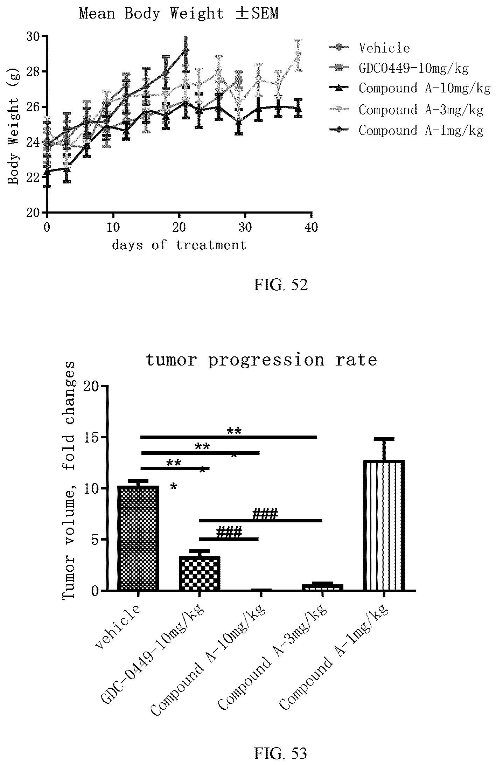

Body weight changes in the treatment groups and the vehicle control group are shown in Table 6-3 and Figure 52. These data indicate that Compound A and GDC-0449 had no significant impact on the body weight of tumor-bearing mice during the treatment period. No animals died accidentally during the entire administration phase. These results demonstrate that CB-17/SCID tumor-bearing mice tolerated the administered doses of both Compound A and GDC-0449.

**Table 6-3 Body Weight Changes in the Treatment Groups and Vehicle Control Group**

| Group | Test Compound | Mean Body Weight at Day 0 (g) | Mean Body Weight at Dosing Endpoint^{a} (g) | Body Weight Change Rate (%) |
|---|---|---|---|---|
| 1 | Vehicle | 24.0 ± 1.8 | 27.2 + 1.5 | 13.3 |
| 2 | GDC-0449 (10 mg/kg) | 23.7 ± 2.1 | 25.9 ± 2.0 | 9.3 |
| 3 | Compound A (10 mg/kg) | 22.4 ± 2.1 | 25.5 + 1.7 | 13.8 |
| 4 | Compound A (3 mg/kg) | 24.6 ± 1.8 | 26.7 ± 2.2 | 8.5 |
| 5 | Compound A (1 mg/kg) | 23.9 + 3.1 | 28.0 ± 2.2 | 17.2 |

| | | | | |
|---|---|---|---|---|
| Notes: Data are presented as mean ±SD. a, The vehicle group received the treatment for a total of 12 days, while the other 4 groups received the treatment for a total of 17 days. | | | | |

### 6.5.2 Tumor Volume

The tumor volumes at different time points during the drug administration and withdrawal observation periods in each treatment group and the vehicle control group are shown in Table 6-4. The tumor growth inhibitory effects of each treatment group in CB-17/SCID tumor-bearing mice are shown in Table 6-5 and Figure 53 (The Y-axis represents the Relative Tumor Volume, defined as the fold change in tumor volume post-treatment (Day 18) compared to pre-treatment (Day 0). Error bars in the figure represent the Standard Error of the Mean (SEM). *** P < 0.001 vs. the vehicle group; ### P < 0.001 vs. the 10 mg/kg GDC-0449 treatment group). Comparing the changes in tumor volume before and after treatment, tumors in the vehicle control group grew very rapidly, the volume had reached more than 10 times that of pre-treatment on day 12. The experiment of this group was terminated due to the excessive tumor burden. Compared to the vehicle control group, all treatment groups significantly inhibited tumor growth. Furthermore, both the high-dose (10 mg/kg) and medium-dose (3 mg/kg) groups of Compound A demonstrated significantly superior anti-tumor efficacy compared to the high-dose GDC-0449 (10 mg/kg) group after 17 days of treatment. The low dose of Compound A (1 mg/kg) did not show a significant inhibitory effect. At the end of the administration, the Relative Tumor Volume in this group was not significantly lower than that in the vehicle control group (P = 0.13).

**Table 6-4 Tumor Volume Changes (mm³) in the Different Groups of Tumor-Bearing Mice During Treatment**

| Days After Grouping | Vehicle | GDC-0449 (10 mg/kg) | Compound A (10 mg/kg) | Compound A (3 mg/kg) | Compound A (1 mg/kg) |
|---|---|---|---|---|---|
| 0 | 216.9 + 51.3 | 244.2 ± 25.4 | 234.5 ± 33.6 | 244.1 + 17.1 | 220.5 ± 43.7 |
| 3 | 330.1+ 114.8 | 255.1 ± 31.8 | 187.9 + 44.7 | 205.3 + 26.0 | 244.9 + 82.4 |
| 6 | 604.6 + 156.1 | 373.8 ± 78.3 | 98.7 ± 28.9 | 147.7 ± 56.5 | 483.8 + 252.8 |
| 9 | 1317.2 ± 411.0 | 397.0 + 83.6 | 47.9 + 29.8 | 140.1 + 62.3 | 759.7 + 401.1 |
| 12 | 2227.7 + 561.6 | 450.0 + 101.5 | 24.5 + 14.9 | 112.7 ± 46.9 | 1234.4 ± 582.9 |
| 15 | - | 653.1 ± 222.2 | 10.9 ± 5.1 | 125.4 ± 58.9 | 2056.6 + 1094.9 |
| 18 | - | 805.3 ± 299.3 | 8.8 + 6.1 | 150.4 + 77.2 | 2868.8 ± 1372.9 |
| 21 | - | 1003.0 + 327.8 | 14.7 + 10.1 | 199.3 ± 93.7 | 4176.0 ± 1871.1 |
| 23 | - | 1433.9 + 514.4 | 16.6 ± 9.7 | 271.2 ± 131.7 | - |
| 26 | - | 2187.9 + 666.9 | 18.1 + 11.5 | 604.1 + 328.5 | - |
| 29 | - | 3384.4 + 894.4 | 43.9 + 37.3 | 887.6 + 407.9 | - |
| 32 | - | - | 92.2 + 101.2 | 1209.2+ 513.6 | - |
| 35 | - | - | 214.8 + 254.9 | 1645.0 ±560.6 | - |
| 38 | - | - | 347.4 + 445.9 | 2352.0 +963.1 | - |

| | | | | | |
|---|---|---|---|---|---|
| Note: Data are presented as mean ± standard deviation. | | | | | |

**Table 6-5 Anti-tumor Efficacy of Compound A and GDC-0449 in SCID Mice with Subcutaneous Tumors**

| Group | Compound | Tumor Volume at Day 0 (mm³) | Tumor Volume at Dosing Endpoint^{a} (mm³) | T/C (%) | TGI^{c} (%) | Fold Change in Tumor Volume | P-value^{d} | P-value |
|---|---|---|---|---|---|---|---|---|
| 1 | Vehicle | 216.9 + 51.3 | 2227.7 + 561.6^{b} | - | - | 10.28 + 1.10 | - | - |
| 2 | GDC-0449 (10 mg/kg) | 244.2 + 25.4 | 805.3 + 299.3 | 32.49 | 67.51 | 3.34 + 1.35 | < 0.0001 | - |
| 3 | Compound A (10 mg/kg) | 234.5 + 33.6 | 8.8 ± 6.1 | 0.39 | 99.61 | 0.04 + 0.03 | < 0.0001 | < 0.0001 |
| 4 | Compound A (3 mg/kg) | 244.1 + 17.1 | 150.4 + 77.2 | 6.03 | 93.97 | 0.62 + 0.33 | < 0.0001 | < 0.0001 |
| 5 | Compound A (1 mg/kg) | 220.5 + 43.7 | 2868.8 + 1372.9 | 124.5 | - | 12.80 + 4.95 | 0.13 | - |

| | | | | | | | | |
|---|---|---|---|---|---|---|---|---|
| Notes: Data are presented as mean ± standard deviation. a, The treatment period was 17 days; tumor volume at dosing endpoint was the volume on Day 18. b, For the vehicle control group, tumor volume was measured on Day 12. After 12 days of vehicle administration, the experimental for this group was terminated due to excessive tumor burden. c, TGI (Tumor Growth Inhibition rate) = [1 - RTV(treatment group) / RTV(control group)] × 100%; RTV: Relative Tumor Volume. d, The significant differences between each treatment group and the solvent control group were compared using Student's t-test. The P value was calculated based on the relative tumor volume after administration. e, The significant differences between each compound A treatment group and the GDC-0449 treatment group were compared using Student's t-test. The P value was calculated based on the relative tumor volume after administration. | | | | | | | | |

### 6.5.3 Tumor Growth Volume

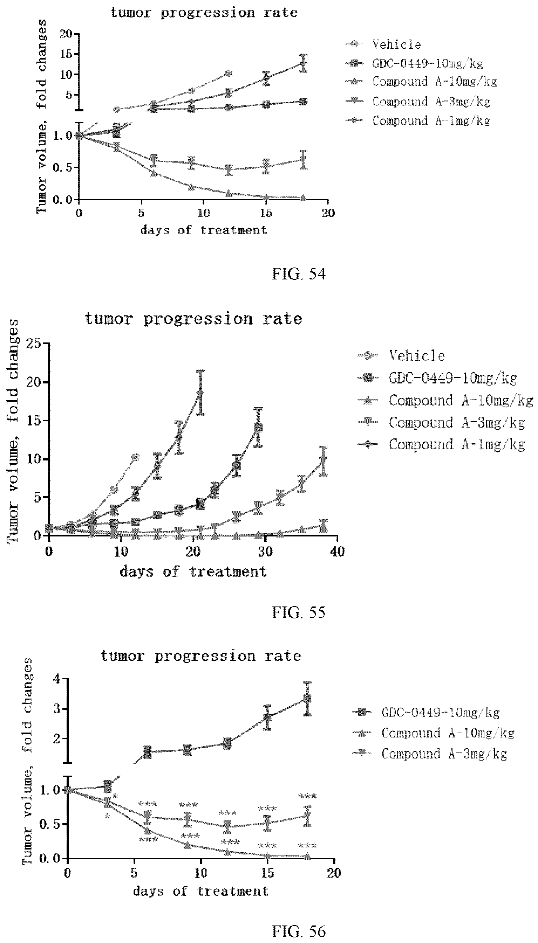

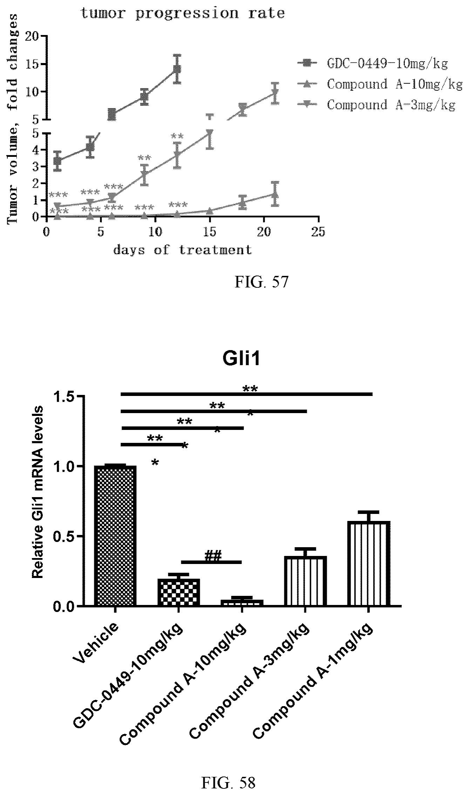

Figures 54 and 55 respectively show the time-course curves of relative tumor volume in CB-17/SCID mice with subcutaneous tumors during the treatment period or the treatment and post-treatment observation period. Figure 56 presents a statistical analysis comparing the relative tumor volume at each time point during the treatment among the Compound A high-dose (10 mg/kg) group, the medium-dose (3 mg/kg) group, and the GDC-0449 high-dose (10 mg/kg) group (*P < 0.05 vs. GDC-0449 10 mg/kg group; ***P < 0.001 vs. GDC-0449 10 mg/kg group). The results demonstrate that both the high and medium-dose groups of Compound A showed significantly superior tumor growth inhibition throughout the entire treatment period compared to the GDC-0449 high-dose (10 mg/kg) group. Figure 57 provides a statistical analysis of tumor recurrence during the post-treatment observation period: (**P < 0.01 vs. GDC-0449 10 mg/kg group; ***P < 0.001 vs. GDC-0449 10 mg/kg group; on the 12th day after discontinuation, the 10 mg/kg GDC-0449 group was terminated due to excessive tumor volume). The results indicate that the recurrent tumor volume in both medium and high-dose group of Compound A is also significantly lower than that of the GDC-0449 group at the same time.

### 6.5.4 Sample Collection

After the treatment period and the observation period of the SCID subcutaneous tumor-bearing mice with the medulloblastoma xenograft model, tumor samples were collected and stored at -80°C.

The results demonstrated that both Compound A (fumarate salt Form F of Compound (I)) and GDC-0449 exhibited high safety profiles, CB-17/SCID tumor-bearing mice tolerated the administered doses well. Throughout the administration period, the mice showed steady weight gain. At specific doses, both Compound (I) fumarate Form F and GDC-0449 significantly inhibited subcutaneous tumor growth in SCID mice. Notably, the medium and high-dose groups of Compound (I) fumarate Form F demonstrated superior tumor suppression efficacy compared to the high-dose of GDC-0449 group. During the drug withdrawal observation period, the recurrence of tumors in the high-dose group of the fumarate crystal form F of compound (I) was later than that of the GDC-0449 group, and the tumor volume of the highdose and medium-dose groups of the fumarate crystal form F of compound (I) was significantly smaller than that of the GDC-0449 group.

### Example 7: Analysis of Shh Signaling Pathway in Tumor Tissues

### 7.1 Experimental Materials

Drug GDC-0449 and compound A are referenced from Example 6.

Mouse strain, age, sex, housing environment, and conditions are referenced from Example 6.

The preparation of the administration solution is referenced from Example 6.

### 7.2 Experimental Design

As shown in Table 7-1.

**Table 7-1 The Grouping and Drug Administration Plan**

| Group | Number of Mice | Drug | Adminidtration Dosage (mg/kg) | Route of Administration | Dosage Regimen |
|---|---|---|---|---|---|
| 1 | 5 | Vehicle | 0 | i.g. | BID × 3 days |
| 2 | 5 | GDC-0449 | 10 | i.g. | BID × 3 days |
| 3 | 5 | Compound A (10 mg/kg) | 10 | i.g. | BID × 3 days |
| 4 | 5 | Compound A (3 mg/kg) | 3 | i.g. | BID × 3 days |
| 5 | 5 | Compound A (1 mg/kg) | 1 | i.g. | BID × 3 days |

| | | | | | |
|---|---|---|---|---|---|
| Note: 1. The dosage is adjusted based on body weight (administration volume = 10 mL/kg); | | | | | |

### 7.3 Experimental Methods

### 7.3.1 Model Establishment

Primary tumor cells were extracted from Math1-Cre/Ptch1 ^{C/C} mice with spontaneous medulloblastoma and subcutaneously inoculated into CB-17/SCID mice to establish a xenograft model of medulloblastoma.

### 7.6.2 Grouping and Drug Administration

CB-17/SCID mice were subcutaneously injected with primary extracted medulloblastoma cells. After 3-4 weeks, a subset of mice developed tumors with volumes ranging from 150 to 300 mm³.

From these mice, 25 tumor-bearing mice with uniform tumor sizes (with CV < 30%, CV = SD/Mean) were selected and randomly divided into 5 experimental groups (Vehicle, GDC-0449-10 mg/kg, Compound A-10 mg/kg, Compound A-3 mg/kg, Compound A-1 mg/kg) according to Table 2. Each group consisted of 5 mice per cage. Administration began on the day after grouping. The administration period was 3 days, with a frequency of BID. Tumor tissues were collected 2 hours after the last administration, and the mRNA expression level of the Glil gene in the tumor tissues was analyzed by RT-qPCR.

### 7.6.3 Observation and tumor volume measurement

During routine examinations, animals were monitored for tumor growth, activity level, food intake, water consumption, weight gain or loss, eye, fur, and any other abnormal behaviors. Animal deaths and clinical abnormalities were recorded.

The tumor tissues were lysed by RNAiso Plius (TaKaRa, 108-95-2) to extractand RNA was then extracted using chloroform extraction and isopropanol precipitation methods. Subsequently, the mRNA level of the Glil gene was detected using a real-time fluorescence quantitative PCR instrument (ABI 7500), with GAPDH serving as the internal reference for quantitative analysis.

### 7.6.4 Discontinuation and Termination Criteria for the Experiment

Treatment was discontinued when the animals' weight decreases by 15% compared to the initial administration or when the animals' condition deteriorates significantly. If the animals' weight subsequently recovered to within a 10% loss compared to baseline, whether to continue the administration was based on the animals' condition. The in vivo experimental for an individual animal or the entire group would be terminated under the following conditions. The animals will be euthanized before death or coma.
➢ If the animals' health condition deteriorated continuously, causing persistent suffering and is unable to eat or drink.
➢ If the animal became emaciated, with a body weight loss exceeding 20% compared to the initial administration.
➢ If the tumor volume of an individual mouse exceeded 3000 mm³ or the mean tumor volume of the entire group exceeded 2000 mm³. 7.6.5 Data Analysis

Experimental data in all tables are presented as "mean ± standard deviation" with error bars in figures representing standard error of the mean (SEM). Student's t-test was used to compare statistical differences in Glil gene mRNA expression levels in tumor tissues among treatment groups. All data were analyzed using GraphPad Prism 5.0. P-value <0.05 was considered statistically significant. ***, P<0.001; ##, P<0.01.

### 7.7 Experimental Results

### 7.7.2 The mRNA Expression Levels of Glil Gene in Tumor Tissues

The mRNA expression levels of Glil gene in tumor tissues of the treatment group and and the vehicle control group, along with statistical analysis, are shown in Table 7-2 and Figure 58.

**Table 7-2 The mRNA Expression Levels of Gli1 Gene in Tumor Tissues of each treatment group**

| Group | Treatment | mRNA Expression Level of Glil Gene | P value ^{a} | P value *^{b}* |
|---|---|---|---|---|
| 1 | Vehicle Control | 1.005 ± 0.004 | - | - |
| 2 | GDC-0449 (10 mg/kg) | 0.194 ± 0.074 | <0.0001 | - |
| 3 | Compound A (10 mg/kg) | 0.045 ± 0.035 | <0.0001 | 0.0034 |
| 4 | Compound A (3 mg/kg) | 0.359 ± 0.113 | <0.0001 | - |
| 5 | Compound A (1 mg/kg) | 0.609 ± 0.140 | 0.0001 | - |

| | | | | |
|---|---|---|---|---|
| Note: The data are presented as "mean ± standard deviation"; a. Use Student's t-test to compare the significant differences between each treatment group and the vehicle control group. b. Use Student's t-test to compare the significant differences between each compound A treatment group and the GDC-0449 treatment group. | | | | |

### 7.8 Results Analysis and Discussion

Glil is a target gene of the Hedgehog signaling pathway, and its mRNA expression level reflects the activation status of the hedgehog signaling pathway. In this experiment, the mRNA expression level of Glil was used as an indicator to compare the inhibition effects of Compound A and GDC-0449 on the Hedgehog signaling pathway.

During the administration period, no abnormal behaviors or animal deaths were observed in SCID tumor-bearing mice treated with either Compound A or GDC-0449. After treatment completion, tumor tissues were collected and the mRNA expression level of Glil was detected and statistically analyzed. The results demonstrated that all treatment groups significantly inhibited Hedgehog signaling compared to the vehicle control group; and the high-dose group of Compound A (10 mg/kg) showed a significantly stronger inhibition than the high-dose group of GDC-0449 (10 mg/kg) (P=0.0034).

This application merely illustrates some specific implementations set forth in this application, where the technical features described in one or more technical solutions may be combined with any one or more technical solutions, and the technical solutions obtained through combination are also within the protection scope of this application, and the technical solutions obtained by combining these solutions have been specifically described in the content disclosed in this application.

## Claims

1. A pharmaceutically acceptable salt of a compound of formula (I): 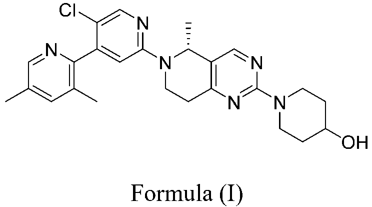 wherein, the salt includes at least one of fumarate, succinate and hydrochloride.

2. The pharmaceutically acceptable salt of the compound of formula (I) according to claim 1, wherein in the fumarate, the molar ratio of the compound of formula (I) to the fumaric acid is 1: 0.2~1, preferably 1: 0.3~0.6, more preferably 1: 0.5.

3. Fumarate of the compound of formula (I) in crystal form:

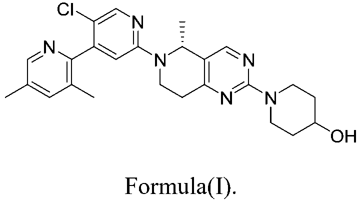

4. The fumarate of the compound of formula (I) according to claim 3, which is a fumarate crystal form A of the compound of formula (I), the x-ray powder diffraction pattern thereof has characteristic peaks at 20 values of 6.0 ± 0.2°, 12.9 ± 0.2°, and 16.2 ± 0.2°; preferably, it further meets at least one of the following conditions:
i. the x-ray powder diffraction pattern has characteristic peaks at one or two or three of a 20 value of 13.3 ± 0.2°, 15.4 ± 0.2°, 19.0 ± 0.2°;
ii. the x-ray powder diffraction pattern has characteristic peaks at one or two or three of a 2θ value of 12.2±0.2°,18.1±0.2°,20.7±0.2°;
iii. the x-ray powder diffraction pattern thereof is basically as shown in FiG.3, and/or has a diffraction peak as shown in Table 2-1;
iv. the molar ratio of the compound (I) to fumaric acid is 1: 0.5;
v. it is anhydrous.

5. The method for preparing the fumarate salt of the compound of formula (I) according to claim 4, wherein the method comprises: stirring the compound of formula (I) and fumaric acid in a mixed solvent of an alkyl ketone and an alkane, filtering and drying to obtain.

6. The fumarate salt of the compound of formula (I) according to claim 3, which is a fumarate crystal form B of the compound of formula (I), the x-ray powder diffraction pattern thereof has characteristic peaks at 20 values of 13.2 ± 0.2°, 16.0 ± 0.2°and 18.6 ± 0.2°; preferably, it further meets at least one of the following conditions:
i. the x-ray powder diffraction pattern has characteristic peaks at one or two or three of a 20 value of 12.7±0.2°, 15.4±0.2°, 16.6±0.2°;
ii. the x-ray powder diffraction pattern has characteristic peaks at one or two or three of a 2θ value of 17.2±0.2°, 18.0±0.2°, 20.4±0.2°;
iii. the x-ray powder diffraction pattern thereof is basically as shown in FiG.6, and/or has a diffraction peak as shown in Table 2-2;
iv. the molar ratio of the compound (I) to fumaric acid is 1: 0.6;
v. it is anhydrous.

7. The method for preparing the fumarate salt of the compound of formula (I) according to claim 6, wherein the method comprises: stirring the compound of formula (I) and fumaric acid in a mixed solvent of an ester and an alkane, and filtering and drying to obtain.

8. The fumarate salt of the compound of formula (I) according to claim 3, which is a fumarate crystal form C of the compound of formula (I), the x-ray powder diffraction pattern thereof has characteristic peaks at 2θ values of 15.1±0.2°, 17.1±0.2° and 19.4±0.2°; preferably, it further meets at least one of the following conditions:
i. the x-ray powder diffraction pattern has characteristic peaks at one or two or three of a 20 value of 11.0±0.2°, 17.8+0.2°, 21.7±0.2°;
ii. the x-ray powder diffraction pattern has characteristic peaks at one or two or three of a 2θ value of 5.0±0.2°, 10.1±0.2°, 27.1±0.2°;
iii. the x-ray powder diffraction pattern thereof is basically as shown in FiG.10, and/or has a diffraction peak as shown in Table 2-3;
iv. the molar ratio of the compound (I) to fumaric acid is 1: 0.5;
v. it is anhydrous.

9. The method for preparing the fumarate salt of the compound of formula (I) according to claim 8, wherein the method comprises: stirring the compound of formula (I) and fumaric acid, or the fumarate salt of the compound of formula (I) in a linear or branched C2 -C6 nitrile, filtering and drying to obtain.

10. The fumarate salt of the compound of formula (I) according to claim 3, which is a fumarate crystal form D of the compound of formula (I), the x-ray powder diffraction pattern thereof has characteristic peaks at 2θ values of 12.7±0.2°, 17.6±0.2° and 19.1±0.2°; preferably, it further meets at least one of the following conditions:
i. the x-ray powder diffraction pattern has characteristic peaks at one or two or three of a 20 value of 6.3±0.2°, 13.2±0.2°, 16.1±0.2°;
ii. the x-ray powder diffraction pattern has characteristic peaks at one or two or three of a 20 value of 17.2±0.2°, 14.0±0.2°, 20.9±0.2°;
iii. the x-ray powder diffraction pattern thereof is basically as shown in FiG.13, and/or has a diffraction peak as shown in Table 2-4;
iv. the molar ratio of the compound (I) to fumaric acid is 1: 0.5.

11. The method for preparing the fumarate salt of the compound of formula (I) according to claim 10, wherein the method comprises: stirring the fumarate salt of the compound of formula (I) in an ether, or a mixed solvent of a cyclic amide and an aromatic hydrocarbon, and filtering and drying to obtain.

12. The fumarate salt of the compound of formula (I) according to claim 3, which is a fumarate crystal form E of the compound of formula (I), the x-ray powder diffraction pattern thereof has characteristic peaks at 2θ values of 15.2±0.2°, 16.0±0.2° and 22.0±0.2°; preferably, it further meets at least one of the following conditions:
i. the x-ray powder diffraction pattern has characteristic peaks at one or two or three of a 20 value of 12.8±0.2°, 20.5±0.2°, 26.0±0.2°;
ii. the x-ray powder diffraction pattern has characteristic peaks at one or two or three of a 2θ value of 16.8±0.2°, 18.1±0.2°, 19.7±0.2°;
iii. the x-ray powder diffraction pattern thereof is basically as shown in FiG.19, and/or has a diffraction peak as shown in Table 2-5;
iv. the molar ratio of the compound (I) to fumaric acid is 1: 0.5.

13. The method for preparing the fumarate salt of the compound of formula (I) according to claim 12, wherein the method comprises: stirring the fumarate salt of the compound of formula (I) in a mixed solvent of an alkyl alcohol and an alkane, and filtering and drying to obtain.

14. The fumarate salt of the compound of formula (I) according to claim 3, which is a fumarate crystal form F of the compound of formula (I), the x-ray powder diffraction pattern thereof has characteristic peaks at 2θ values of 11.1±0.2°, 16.6±0.2° and 22.4±0.2°; preferably, it further meets at least one of the following conditions:
i. the x-ray powder diffraction pattern has characteristic peaks at one or two or three of a 20 value of 16.9±0.2°, 18.6±0.2°, 21.0±0.2°;
ii. the x-ray powder diffraction pattern has characteristic peaks at one or two or three of a 2θ value of 5.5±0.2°, 14.5±0.2°, 25.0±0.2°;
iii. the x-ray powder diffraction pattern thereof is basically as shown in FiG.23, and/or has a diffraction peak as shown in Table 2-6;
iv. the molar ratio of the compound (I) to fumaric acid is 1: 0.5;
v. it is anhydrous.

15. The method for preparing a fumarate salt of the compound of formula (I) according to claim 14, wherein the method is selected from any one of the following:
i. stirring the compound of formula (I) and fumaric acid in an organic solvent, filtering and drying to obtain; the organic solvent is selected from a mixed solvent of an alkyl ketone and an alkane, C2 -C6 nitrile, an ether, a mixed solvent of an alkyl alcohol and an alkane, and a mixed solvent of an alkyl alcohol and water;
ii. stirring the fumarate salt of the compound of formula (I) in an organic solvent, filtering and drying to obtain. wherein the organic solvent is selected from an alkyl ketone, a C2 -C6 nitrile, and an ether.

16. The fumarate salt of the compound of formula (I) according to claim 3, which is a fumarate crystal form G of the compound of formula (I), the x-ray powder diffraction pattern thereof has characteristic peaks at 2θ values of 15.6±0.2°, 17.7±0.2°, 20.6±0.2°; preferably, it further meets at least one of the following conditions:
i. the x-ray powder diffraction pattern thereof is basically as shown in FiG.27, and/or has a diffraction peak as shown in Table 2-9;
ii. the molar ratio of the compound (I) to fumaric acid is 1:0.3.

17. The method for preparing the fumarate salt of the compound of formula (I) according to claim 16, wherein the method comprises: stirring the fumarate salt of the compound of formula (I) in a mixed solvent of an alkyl alcohol and water, or a mixed solvent of a cyclic amide and water, filtering and drying to obtain.

18. The fumarate salt of the compound of formula (I) according to claim 3, which is a fumarate crystal form H of the compound of formula (I), the x-ray powder diffraction pattern thereof has characteristic peaks at 2θ values of 12.4±0.2°, 16.2±0.2° and 21.8±0.2°; preferably, it further meets at least one of the following conditions:
i. the x-ray powder diffraction pattern has characteristic peaks at one or two or three of a 20 value of 12.8±0.2°, 15.8±0.2°, 18.0±0.2°;
ii. the x-ray powder diffraction pattern has characteristic peaks at one or two or three of a 2θ value of 14.5±0.2°, 16.9±0.2°, 21.2±0.2°;
iii. the x-ray powder diffraction pattern thereof is basically as shown in FiG.30, and/or has a diffraction peak as shown in Table 2-10;
iv. the molar ratio of the compound (I) to fumaric acid is 1: 0.5.

19. The method for preparing the fumarate salt of the compound of formula (I) according to claim 18, wherein the method comprises: stirring a fumarate salt of the compound of formula (I) in a mixed solvent of an alkyl alcohol and an alkane, filtering and drying to obtain.

20. Succinate of the compound of formula (I) in crystal form:

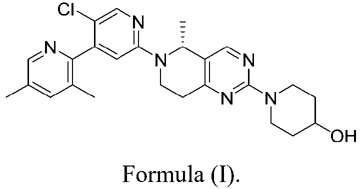

21. The succinate of the compound of formula (I) according to claim 20, which is a succinate crystal form A of the compound of formula (I), the x-ray powder diffraction pattern thereof has characteristic peaks at 2θ values of 22.1 ± 0.2° and 27.2 ± 0.2°;
preferably, the x-ray powder diffraction pattern thereof has a diffraction peak substantially as shown in Table 1-3.

22. The method for preparing the succinate salt of the compound of formula (I) according to claim 21, wherein the method comprises: stirring the compound of formula (I) and succinic acid in ether, filtering and drying to obtain the compound.

23. The succinate of the compound of formula (I) according to claim 20, which is a succinate crystal form B of the compound of formula (I), the x-ray powder diffraction pattern thereof has characteristic peaks at 2θ values of 9.5±0.2°, 14.4±0.2° and 23.6±0.2°; preferably, it further meets at least one of the following conditions:
i. the x-ray powder diffraction pattern has characteristic peaks at one or two or three of a 20 value of 15.1±0.2°, 19.2±0.2°, 25.5±0.2°;
ii. the x-ray powder diffraction pattern has characteristic peaks at one or two or three of a 2θ value of 15.6±0.2°, 22.3±0.2°, 24.0±0.2°;
iii. the x-ray powder diffraction pattern thereof has a diffraction peak as shown in Table 1-4.

24. The method for preparing the succinate salt of the compound of formula (I) according to claim 23, wherein the method comprises: stirring the compound of formula (I) and succinic acid in a mixed solvent of an alkyl ketone and an alkane, and filtering and drying to obtain the compound.

25. A pharmaceutical composition, comprising: (i) the pharmaceutically acceptable salt of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23; and (ii) a pharmaceutically acceptable carrier.

26. A combined application composition, comprising: (i) any one or more of cisplatin, paclitaxel, camptothecin, trastuzumab, glider, imatinib, gefitinib, erlotinib, and lapatinib; and (ii) the pharmaceutically acceptable salts of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23; which are simultaneously or sequentially used.

27. The use of the pharmaceutically acceptable salts of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23, or the pharmaceutical composition according to claim 25, or the combined application composition according to claim 26 in the preparation of a drug, the drug being used for treating a disease caused by abnormal activation of a hedgehog signaling pathway; preferably, the disease is selected from tumor; which includes: basal cell carcinoma, breast cancer, cervical cancer, colon cancer, rectal cancer, glioma, hepatocellular carcinoma, leukemia, lung cancer, lymphoma, neuroblastoma, multiple myeloma, oral cancer, ovarian cancer, pancreatic cancer, prostate cancer, gastric cancer, upper GI cancer, esophageal cancer, nasopharyngeal carcinoma, skin cancer, bone cancer, kidney cancer, sarcoma, and brain cancer.

28. A combined application composition comprising: (i) any one or more of N-acetylcysteine, pirfenidone, nintedanib, glucocorticoids (such as methylprednisolone, prednisone, fluticasone), immunosuppressants (such as cyclosporine, cyclophosphamide, Azathioprine), and endothelin receptor antagonists (such as Macitentan, Bosentan, Ambrisentan ), JNK Inhibitors (Tanzisertib (CC -930)), Itraconazole, Vismodegib, hydroxyurea, Danazole, Amikacin, Dornase alfa, Tobramycin, Tiotropium bromide, Treprostinil, Zileuton, Saridegib (IPI -926); and (ii) the pharmaceutically acceptable salts of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23; which are simultaneously or sequentially used.

29. The use of the pharmaceutically acceptable salts of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23, or the pharmaceutical composition according to claim 25, or the combined application composition according to claim 28 in the preparation of a drug, the drug being used for treating a disease caused by abnormal activation of a hedgehog signaling pathway; preferably, the disease is selected from interstitial pneumonia; more preferably, the disease is selected from idiopathic interstitial pneumonia, such as idiopathic pulmonary fibrosis, desquamative interstitial pneumonia, non-specific interstitial pneumonia, cryptic pneumonia, respiratory bronchiolitis-related interstitial lung diseases, lymphocyte interstitial pneumonia; further preferably, the disease is selected from idiopathic pulmonary fibrosis.

30. A method for inhibiting hedgehog pathway activation or treating a disease caused by abnormal activation of hedgehog signaling pathway or for anti-tumor, comprising administering to a mammalian subject a therapeutically effective amount of at least one of the pharmaceutically acceptable salts of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23, or the pharmaceutical composition according to claim 25, or the combined application composition according to claim 26.

31. A method for inhibiting hedgehog pathway activation or treating a disease caused by abnormal activation of hedgehog signaling pathway or treating interstitial pneumonia, comprising administering to a mammalian subject a therapeutically effective amount of at least one of the pharmaceutically acceptable salts of the compound of formula (I) according to claim 1 or 2, or the fumarate of the compound of formula (I) according to any one of claims 3, 4, 6, 8, 10, 12, 14, 16, 18, or the succinate of the compound of formula (I) according to any one of claims 20, 21, 23, or the pharmaceutical composition according to claim 25, or the combined application composition according to claim 28.
